# EUROPEAN PATENT SPECIFICATION

(11) **EP 1 658 289 B1**
(45) Date of publication and mention of the grant of the patent: **18.03.2009**
(21) Application number: 04781205.2
(22) Date of filing: 16.08.2004
(51) Int. Cl.: C07D 471/04, A61K 31/435, A61P 35/00

(54) **3-SUBSTITUTED IMIDAZOPYRIDINE-DERIVATIVES AS C-KIT INHIBITORS**
3-SUBSTITUIERTE IMIDAZOPYRIDINDERIVATE ALS C-KIT-INHIBITOREN
INHIBITEURS D'UN ENSEMBLE D'IMIDAZOPYRIDINE C N3 SUBSTITUE

(30) Priority: 21.08.2003 US 496776 P
(43) Date of publication of application: 24.05.2006
(73) Proprietor: OSI Pharmaceuticals, Inc., Melville, NY 11747 (US)
(72) Inventor: CASTELHANO, Arlindo, L., Farmingdale, NY 11735 (US); CREW, Andrew, Phillip, Farmingdale, NY 11735 (US); DONG, Han-Qing, Farmingdale, NY 11735 (US); LAUFER, Radoslaw, Farmingdale, NY 11735 (US); LI, An-Hu, Farmingdale, NY 11735 (US); QIU, Li, Farmingdale, NY 11735 (US); SAMBROOK SMITH, Colin, Peter, Oxford OX4 6LT (GB); ZHANG, Tao, Farmingdale, NY 11735 (US)
(74) Representative: Blakey, Alison Jane
(86) International application number: PCT/US2004/026483
(87) International publication number: WO 2005/021544

(56) References cited:
- WO-A-94/10171
- WO-A-03/020698
- CRISTALLI ET AL.: "Purine and 1-Deazapurine Ribonucleosides and Deoxyriboucleosides: Synthesis and Biological Activity" J. MED. CHEM., vol. 34, 1991, pages 2226-2230, XP002323978

## Description

### BACKGROUND OF THE INVENTION

The present invention is directed to N3-substituted imidazopyridine compounds. In particular, the present invention is directed to N3-substituted imidazopyridine compounds that are inhibitors of c-Kit proto-oncogene (also known as KIT, CD-117, stem cell factor receptor, mast cell growth factor receptor).

The c-Kit proto-oncogene is believed to be important in embryogenesis, melanogenesis, hematopoiesis, and the pathogenesis of mastocytosis, gastrointestinal tumors, and other solid tumors, as well as certain leukemias, including AML. Accordingly, it would be desirable to develop novel compounds that are inhibitors of the c-Kit receptor.

Many of the current treatment regimes for hyperproliferative disorders (cancer) utilize compounds that inhibit DNA synthesis. Such compounds' mechanism of operation is to be toxic to cells, particularly to rapidly dividing tumor cells. Thus, their broad toxicity can be a problem to the subject patient. However, other approaches to anti-cancer agents that act other than by the inhibition of DNA synthesis have been explored to try to enhance the selectivity of the anti-cancer action and thereby reduce adverse side-effects.

It is known that a cell may become cancerous by virtue of the transformation of a portion of its DNA into an oncogene (i.e. a gene which, on activation, leads to the formation of malignant tumor cells). Many oncogenes encode proteins that are aberrant protein-tyrosine kinases capable of causing cell transformation. By a different route, the overexpression of a normal proto-oncogenic tyrosine kinase can also result in proliferative disorders, sometimes resulting in a malignant phenotype. Alternatively, co-expression of a receptor tyrosine kinase and its cognate ligand within the same cell type may also lead to malignant transformation.

Receptor tyrosine kinases are large enzymes which span the cell membrane and possess i) an extracellular binding domain for growth factors such as KIT ligand (also known as stem cell factor (SCF, Steel factor (SLF) or mast cell growth factor (MGF)), ii) a transmembrane domain, and iii) an intracellular portion which functions as a kinase to phosphorylate specific tyrosine residues in proteins. Binding of KIT ligand to KIT tyrosine kinase results in receptor homodimerization, the activation of KIT tyrosine kinase activity, and the subsequent phosphorylation of a variety of protein substrates, many of which are effectors of intracellular signal transduction, these events can lead to enhanced cell proliferation or promote enhanced cell survival. With some receptor kinases, receptor heterodimerization can also occur.

It is known that such kinases are frequently aberrantly expressed in common human cancers such as breast cancer, head and neck cancers, gastrointestinal cancer such as colon, rectal or stomach cancer, leukemia, and ovarian, bronchial, lung or pancreatic cancer. KIT kinase expression has been documented in a wide variety of human malignancies such as mastocytosis/ mast cell leukemia, gastrointestinal stromal tumors (GIST), small cell lung carcinoma (SCLC), sinonasal natural killer/T-cell lymphoma, testicular cancer (seminoma), thyroid carcinoma, malignant melanoma, ovarian carcinoma, adenoid cystic carcinoma, acute myelogenous leukemia (AML), breast carcinoma, pediatric T-cell acute lymphoblastic leukemia, angiosarcoma, anaplastic large cell lymphoma, endometrial carcinoma, and prostate carcinoma. The kinase activity of KIT has been implicated in the pathophysiology of several of these - and additional tumors - including breast carcinoma, SCLC, GIST, germ cell tumors, mast cell leukemia, neuroblastoma, AML, melanoma and ovarian carcinoma.

Several mechanisms of KIT activation in tumor cells have been reported, including activating mutations, autocrine and paracrine activation of the receptor kinase by its ligand, loss of protein-tyrosine phosphatase activity, and cross activation by other kinases. The transforming mechanisms initiated by the activating mutations are thought to include dimer formation and increased intrinsic activity of the kinase domain, both of which result in constitutive ligand-independent kinase activation, and possibly altered substrate specificity. More than thirty activating mutations of the Kit protein have been associated with highly malignant tumors in humans.

Accordingly, it has been recognized that inhibitors of receptor tyrosine kinases are useful as selective inhibitors of the growth of mammalian cancer cells. For example, Gleevec^{™} (also known as imatinib mesylate, or STI571), a 2-phenylpyrimidine tyrosine kinase inhibitor that inhibits the kinase activity of the BCR-ABL fusion gene product, was recently approved by the U.S. Food and Drug Administration for the treatment of CML. Gleevec^{™}, in addition to inhibiting BCR-ABL kinase, also inhibits the KIT kinase and PDGF receptor kinases, although it is not effective against all mutant isoforms of the KIT kinase. Kit ligand-stimulated growth of MO7e human leukemia cells is inhibited by Gleevec^{™}, which also induces apoptosis under these conditions. By contrast, GM-CSF stimulated growth of MO7e human leukemia cells is not affected by Gleevec^{™}. Further, in recent clinical studies using Gleevec^{™} to treat patients with GIST, a disease in which KIT kinase is involved in transformation of the cells, many of the patients showed marked improvement.

These studies demonstrate how KIT chase inhibitors can treat tumors whose growth is dependent on KIT kinase activity. Other kinases inhibitors show even greater kinase selectivity. For example, the 4-anilinoquinazoline compound Tarceva^{™} inhibits only EGF receptor kinase with high potency, although it can inhibit the signal transduction of other receptor kinase, probably by virtue of the fact that these receptors heterodimerize with EGF receptor.

Although anti-cancer compounds such as those described above make a significant contribution to the art, there is a continuing need for improved anti-cancer pharmaceuticals, and it would be desirable to develop new compounds with better selectivity or potency, or with reduced toxicity or side effects.

U.S. Patent Nos. 5,990,146 and 6,218,388 describe benzimidazoles for inhibiting protein tyrosine kinase mediated cellular proliferation. U.S. Patent No. 6,348,032 describes method of inhibiting neoplastic cells with benzimidazole derivatives. International Patent Publication No. WO 01/21634 describes benzimidazole derivatives and combinatorial libraries thereof International Patent Publication No. WO 01/57020 describes indole and benzimidazole inhibitors of factor Xa. International Patent Publication No. WO 00/15222 describes fused pyridine inhibitors of cGMP phosphodiesterase. International Patent Publication No. WO 01/12600 describes inhibitors of Factor Xa. International Patent Publication No. WO 97/12613 describes method for treating and preventing inflammation and atherosclerosis.

U.S. Patent No. 6,316,474 describes 2-benzyl and 2-heteroaryl benzimidazole NMDA/NR2b antagonists. U.S. Patent No. 6,479,508 describes viral polymerase inhibitors. U.S. Patent No. 6,444,617 describes fused-heterocycle dicarboxylic acid diamide derivatives or salts thereof, herbicide and usage thereof U.S. Patent Nos. 6,087,380,6,414,008, and 6,469,039 describe disubstituted bicyclic heterocycles. U.S. Patent No. 5,118,688 describes tetrahydropyridoquinolone derivatives. U.S. Patent No. 4,975,435 describes certain 1H-pyrrolo[3,4-b]quinolin-1-one-9-amino-2,3-dihydro derivatives useful for treating anxiety. U.S. Patent No. 6,548,524 describes orthosulfonamido bicyclic heteroaryl hydroxamic acids. U.S. Patent No. 6,348,474 describes sulfonamide compounds.

U.S. Patent Nos. 5,972,980 and 6,001,866 describe method for treating and preventing inflammation and atherosclerosis. U.S. Patent No. 5,814,651 describes catechol diethers as selective PDEIV inhibitors. U.S. Patent No. 6,329,383 describes 2-amino-5-pyrimidine acetic acid compounds. U.S. Patent No. 5,688,809 describes 5-heteroarylindole derivatives. European Patent Application No. EP 0 846 689 describes benzimidazole compounds. International Patent Publication No. WO 00/59888 describes N-benzimidazolylmethyl- and N-indolylmethyl-benzamides and their use as CRF modulators. International Patent Publication No. WO 02/069965 describes benzimidazole derivatives as therapeutic agents. International Patent Publication No. WO 02/30886 describes heterocyclic angiogenesis inhibitors. U.S. Patent No. 6,162,804 describes tyrosine kinase inhibitors. U.S. Patent No. 6,465,484 describes angiogenesis inhibitors. International Patent Publication No. WO 00/12089 describes novel angiogenesis inhibitors.

German Patent Publication No. DE 2244908 describes selectively permeable polymeric membranes. European Patent Application No. EP 0 706 795 describes catechol diether compounds as inhibitors of TNF release. International Patent Publication No. WO 02/076960 describes transition metal mediated process. International Patent Publication No. WO 02/059118 describes process for N-(oxyalkylation) of carboxamides. International Patent Publication No. WO 02/04425 describes viral polymerase inhibitors. International Patent Publication No. WO 02/083143 describes CXCR3 antagonists. International Patent Publication No. WO 01/57019 describes indolone and benzimidazolone inhibitors of factor Xa. European Patent Application No. EP 1 085 372 describes photographic material having improved color reproduction. International Patent Publication No. WO 01/14342 describes aminocarbonyl-substituted benzimidazole derivatives. International Patent Publication No. WO 00/76501 describes IL-8 receptor antagonists. Cristalli et al, J. Med. Chem., 1991, 34, 2226-2230 disclose purine and 1-deazapurine nucleoside derivatives some of which are cytotoxic agents.

Thus, it is desirable to develop compounds that exhibit Kit inhibition in order to treat oncology. Further, such compounds may be active in other kinases such as, for example, GIST, FLT3, Hematopoietic R-PTKs, PDGFR-beta or KDR to add efficacy in mast cell leukemias, small cell lung cancer (SCLC), mastocytosis, leukemias, myelodysplastic disorders, or angiogenic dependent diseases.

### SUMMARY OF THE INVENTION

Compounds represented by Formula (I): 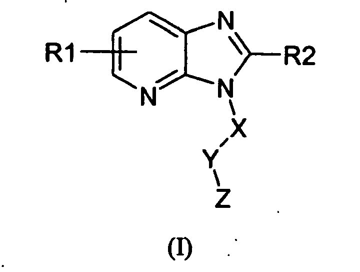 or a pharmaceutically acceptable salt or N-oxide thereof, are useful in the treatment of tumors.

### DETAILED DESCRIPTION OF THE INVENTION

The present invention is directed to a compound represented by Formula (I) 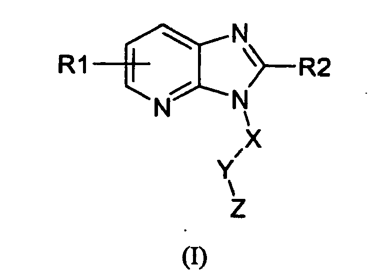 wherein:

R1 is -CO-NR₃R₃₁;

R2 is H, -C₀₋₈alkyl, or -C₃₋₁₀cycloalkyl;

X is a cyclyl or heterocyclyl group optionally substituted with 1 or more substituents chosen from H, halogen, NR₃₂R₃₃, NR₃₂COR₃₃, NR₃₂CO2R₃₃, NR₃₂SO₂R₃₃ OR₃₂, SR₃₂, SO₂R₃₂, SO₂NR₃₂R₃₃, CO₂R₃₂, CO₂H, CONR₃₂R₃₃, -C₀₋₈alkyl, -C₂₋₈alkenyl, -C₂₋₈alkynyl, CN, CF₃, OCF₃, NO₂, oxo, cyclyl or a heterocyclyl group;

Y is absent, or 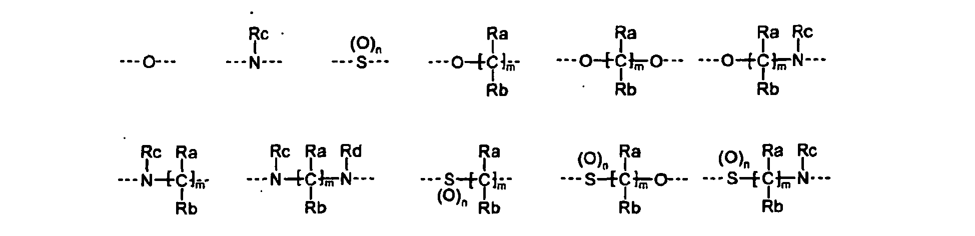 or

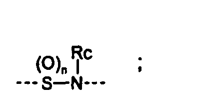

wherein the point of attachment to X can be from either the left or the right of the linkers as shown;

Rₐ and R_{b} each independently is -C₀₋₈alkyl, -C₂₋₈alkenyl, -C₂₋₈alkynyl, -C₃₋₁₀cycloalkyl, -C₃₋₁₀cycloalkenyl, -C₁₋₈alkoxy, -thioC₁₋₈alkyl, carboxyl, -N(C₀₋₈alkyl)(C₀₋₈alkyl), oxo, or hydroxy; or taken together with the C to which they are attached, form a saturated or partially unsaturated 3-10 membered ring optionally containing 0-4 N, O, S, SO, or SO₂ at the ring nodes;

R_{c} and R_{d} each independently is -C₀₋₈alkyl, -C₂₋₈alkenyl, benzyl, or acyl; or taken together, or with Rₐ or R_{b}, form a 3-7 membered saturated or partially unsaturated ring;

m is 0, 1, 2, 3, 4, or 5;

Z is a cyclyl or heterocyclyl group, optionally substituted with 1 or more substituents chosen from halogen, NR₃₄R₃₅, NR₃₄COR₃₅, NR₃₄CO2R₃₅, NR₃₄SO₂R₃₅, OR₃₄, SR₃₄, SO₂R₃₄, SO₂NR₃₄R₃₅, CO₂R₃₄, CO₂H, CONR₃₄R₃₅, -C₀₋₈alkyl, -C₂₋₈alkenyl, -C₂₋₈alkynyl, CN, CF₃, NO₂, oxo, cyclyl or a heterocyclyl group; or, when X and Y are present, Z can be -C₁₋₈alkyl or -C₁₋₈alkyl-O-C₁₋₈alkyl; and

R₃, R₃₁, R₃₂, R₃₃, R₃₄ and R₃₅ are independently C₀₋₈alkyl optionally substituted with a heterocyclyl or OH substituent; -C₀₋₈alkyl-C₃₋₈cycloalkyl, CF₃, -C₀₋₈alkyl-O-C₀₋₈alkyl, -C₀₋₈alkyl-N(C₀₋₈alkyl)(C₀₋₈alkyl), -C₀₋₈alkyl-S(O)₀₋₂-C₀₋₈alkyl; or heterocyclyl optionally substituted with -C₀₋₈alkyl, cyclyl or substituted cyclyl substituent;

wherein cyclyl groups represent 3-10 membered aromatic, partially aromatic or non-aromatic cyclyl groups, and heterocyclyl groups represent 3-10 membered aromatic, partially aromatic or non-aromatic heterocyclyl groups containing one or more atoms chosen independently from N, O and S and their oxides;
or a pharmaceutically acceptable salt or N-oxide thereof.

In an embodiment, the present invention is directed to a compound represented by Formula (I), or a pharmaceutically acceptable salt or N-oxide thereof, wherein R1 is -CONR₃R₃₁; X is cyclyl; and the other variables are as described above for Formula (I).

In another embodiment, the present invention is directed to a compound represented by Formula (I), or a pharmaceutically acceptable salt or N-oxide thereof, wherein R1 is -CONR₃R₃₁; X is cyclyl; Y is absent; and the other variables are as described above for Formula (I).

In yet another embodiment, the present invention is directed to a compound represented by Formula (I), or a pharmaceutically acceptable salt or N-oxide thereof, wherein R1 is -CONR₃R₃₁; X is cyclyl; Y is 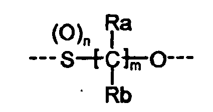 and the other variables are as described above for Formula (I).

In yet another embodiment, the present invention is directed to a compound represented by Formula (I), or a pharmaceutically acceptable salt or N-oxide thereof, wherein R1 is -CONR₃R₃₁ X is cyclyl; Y is 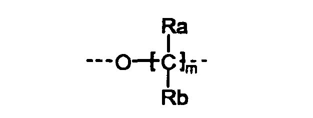 and the other variables are as described above for Formula (I).

In yet another embodiment, the present invention is directed to a compound represented by Formula (I), or a pharmaceutically acceptable salt or N-oxide thereof, wherein R1 is -CONR₃R₃₁; X is cyclyl; Y is 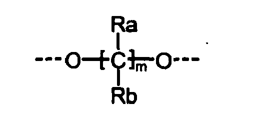 and the other variables are as described above for Formula (I).

In yet another embodiment, the present invention is directed to a compound represented by Formula (I), or a pharmaceutically acceptable salt or N-oxide thereof, wherein R1 is -CONR₃R₃₁; X is cyclyl; Y is 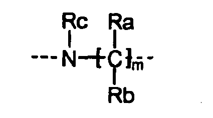 and the other variables are as described above for Formula (I).

As used herein, unless stated otherwise, "alkyl" as well as other groups having the prefix "alk" such as, for example, alkoxy, alkanyl, alkenyl, alkynyl, and the like, means carbon chains which may be linear or branched or combinations thereof. Examples of alkyl groups include methyl, ethyl, propyl, isopropyl, butyl, sec- and tert-butyl, pentyl, hexyl, heptyl and the like. "Alkenyl", "alkynyl" and other like terms include carbon chains having at least one unsaturated carbon-carbon bond.

As used herein, "C₀₋₄alkyl" is used to mean an alkyl having 0-4 carbons - that is, 0, 1, 2, 3, or 4 carbons in a straight or branched configuration. An alkyl having no carbon is hydrogen when the alkyl is a terminal group. An alkyl having no carbon is a direct bond when the alkyl is a bridging (connecting) group.

The terms "cycloalkyl", "carbocyclic ring", cyclic", or "cyclyl" mean 3-10 membered mono or polycyclic aromatic, partially aromatic or non-aromatic ring carbocycles containing no heteroatoms, and include mono-, bi-, and tricyclic saturated carbocycles, as well as fused and bridged systems. Such fused ring systems can include one ring that is partially or fully unsaturated, such as a benzene ring, to form fused ring systems, such as benzofused carbocycles. Cycloalkyl includes such fused ring systems as spirofused ring systems. Examples of cycloalkyl and carbocyclic rings include C₃₋₈cycloalkyl such as cyclopropyl, cyclobutyl, cyclopentyl, cyclohexyl, and decahydronaphthalene, adamantane, indanyl, 1,2,3,4-tetrahydronaphthalene and the like.

The term "halogen" includes fluorine, chlorine, bromine, and iodine atoms.

The term "carbamoyl" unless specifically described otherwise means -C(O)-NH- or NH-C(O)-.

The term "aryl" is well known to chemists. The preferred aryl groups are phenyl and naphthyl.

The term "hetaryl" is well known to chemists. The term includes 5- or 6-membered heteroaryl rings containing 1-4 heteroatoms chosen from oxygen, sulfur, and nitrogen in which oxygen and sulfur are not next to each other. Examples of such heteroaryl rings are furyl, thienyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyridyl, pyridazinyl, pyrimidinyl, pyrazinyl, and triazinyl. The term "hetaryl" includes hetaryl rings with fused carbocyclic ring systems that are partially or fully unsaturated, such as a benzene ring, to form a benzofused hetaryl. For example, benzimidazole, benzoxazole, benzothiazole, benzofuran, quinoline, isoquinoline, quinoxaline, and the like.

Unless otherwise stated, the terms "heterocyclic ring", "heterocycle", "heterocyclic", and "heterocyclyl" are equivalent, and are defined as for cyclic but also contain one or more atoms chosen independently from N, O, and S and their oxides, provided such derivatives exhibit appropriate and stable valencies and excludes moieties containing O-O, S(O)ₙ-S(O)ₙ, S(O)ₙ-O bonds where n=0-2. The terms include 4-8-membered saturated rings containing one or two heteroatoms chosen from oxygen, sulfur, and nitrogen. Examples of heterocyclic rings include azetidine, oxetane, tetrahydrofuran, tetrahydropyran, oxepane, oxocane, thietane, thiazolidine, oxazolidine, oxazetidine, pyrazolidine, isoxazolidine, isothiazolidine, tetrahydrothiophene, tetrahydrothiopyran, thiepane, thiocane, azetidine, pyrrolidine, piperidine, azepane, azocane, [1,3]dioxane, oxazolidine, piperazine, homopiperazine, morpholine, thiomorpholine, and the like. Other examples of heterocyclic rings include the oxidized forms of the sulfur-containing rings. Thus, tetrahydrothiophene-1-oxide, tetrahydrothiophene-1,1-dioxide, thiomorpholine-1-oxide, thiomorpboline-1,1-dioxide, tetrahydrothiopyran-1-oxide, tetrahydrothiopyran-1,1-dioxide, thiazolidine-1-oxide, and thiazolidine-1,1-dioxide are also considered to be heterocyclic rings. The term "heterocyclic" also includes fused ring systems and can include a carbocyclic ring that is partially or fully unsaturated, such as a benzene ring, to form benzofused heterocycles. For example, 3,4-dihydro-1,4-benzodioxine, tetrahydroquinoline, tetrahydroisoquinoline and the like.

Compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof.

The above Formula (I) is shown without a definitive stereochemistry at certain positions. The present invention includes all stereoisomers of Formula (I) and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

The invention also encompasses a pharmaceutical composition that is comprised of a compound of Formula (I) in combination with a pharmaceutically acceptable carrier.

Preferably, the composition is comprised of a pharmaceutically acceptable carrier and a non-toxic therapeutically effective amount of a compound of Formula (I) as described above (or a pharmaceutically acceptable salt or N-oxide thereof).

Moreover, within this preferred embodiment, the invention encompasses a pharmaceutical composition for the treatment of disease by the inhibition of the c-Kit kinase, which may be a wild-type or mutant form of the protein, comprising a pharmaceutically acceptable carrier and a non-toxic therapeutically effective amount of compound of Formula (I) as described above (or a pharmaceutically acceptable salt or N-oxide thereof).

The compounds and compositions of the present invention are effective for treating mammals such as, for example, humans.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particularly preferred are citric, hydrobromic, hydrochloric, maleic, phosphoric, sulfuric, methanesulfonic, and tartaric acids.

The pharmaceutical compositions of the present invention comprise a compound represented by Formula (I) (or a pharmaceutically acceptable salt or N-oxide thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula (I), or pharmaceutically acceptable salts or N-oxides thereof, of this invention can be combined as the active ingredient in intimate admixture with a pharmaceutical carrier according to conventional pharmaceutical compounding techniques. The carrier may take a wide variety of forms depending on the form of preparation desired for administration. E.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound represented by Formula (I), or a pharmaceutically acceptable salt or N-oxide thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier and a compound or a pharmaceutically acceptable salt or N-oxide of Formula (I). The compounds of Formula (I), or pharmaceutically acceptable salts or N-oxides thereof, can also be included in pharmaceutical compositions in combination with one or more other therapeutically active compounds.

The pharmaceutical compositions of this invention include a pharmaceutically acceptable liposomal formulation containing a compound of Formula (I) or a pharmaceutically acceptable salt or N-oxide thereof.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent or other such excipient. These excipients may be, for example, inert diluents such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example, magnesium stearate, stearic acid or talc. The tablets may be uncoated or they may be coated by known techniques to delay disintegration and absorption in the gastrointestinal tract and thereby provide a sustained action over a longer time. For example, a time delay material such as glyceryl monostearate or glyceryl distearate may be used.

In hard gelatin capsules, the active ingredient is mixed with an inert solid diluent, for example, calcium carbonate, calcium phosphate or kaolin. In soft gelatin capsules, the active ingredient is mixed with water or an oil medium, for example, peanut oil, liquid paraffin or olive oil. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient.

For example, a formulation intended for the oral administration to humans may contain from about 0.5mg to about 5g of active agent, compounded with an appropriate and convenient amount of carrier material, which may vary from about 5 to about 95 percent of the total composition. Unit dosage forms will generally contain between from about 1mg to about 2g of the active ingredient, typically 25mg, 50mg, 1 00mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula (I) of this invention, or a pharmaceutically acceptable salt or N-oxide thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound described by Formula (I), or pharmaceutically acceptable salts or N-oxides thereof, may also be prepared in powder or liquid concentrate form.

Generally, dosage levels on the order of from about 0.01 mg/kg to about 750mg/kg of body weight per day are useful in the treatment of the above-indicated conditions, or alternatively about 0.5mg to about 75g per patient per day. For example, breast cancer, head and neck cancers, and gastrointestinal cancer such as colon, rectal or stomach cancer may be effectively treated by the administration of from about 0.01 to 500mg of the compound per kilogram of body weight per day, or alternatively about 0.5mg to about 50g per patient per day.

Similarly, leukemia, ovarian, bronchial, lung, and pancreatic cancer may be effectively treated by the administration of from about 0.01 to 500mg of the compound per kilogram of body weight per day, or alternatively about 0.5mg to about 50g per patient per day.

Mastocytosis/ mast cell leukemia, gastrointestinal stromal tumors (GIST), small cell lung carcinoma (SCLC), colon cancer, sinonasal natural killer/T-cell lymphoma, testicular cancer (seminoma), thyroid carcinoma, malignant melanoma, ovarian carcinoma, adenoid cystic carcinoma, acute myelogenous leukemia (AML), breast carcinoma, pediatric T-cell acute lymphoblastic leukemia, angiosarcoma, anaplastic large cell lymphoma, endometrial carcinoma, and prostate carcinoma may be effectively treated by the administration of from about 0.01 to 500mg of the compound per kilogram of body weight per day, or alternatively about 0.5mg to about 50g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of the present invention, or pharmaceutically acceptable salts or N-oxides thereof, can also be effectively administered in conjunction with other cancer therapeutic compounds. For example, cytotoxic agents and angiogenesis inhibiting agents can be advantageous co-agents with the compounds of the present invention. Accordingly, the present invention includes compositions comprising the compounds represented by Formula (I), or a pharmaceutically acceptable salt or N-oxide thereof, and a cytotoxic agent or an angiogenesis-inhibiting agent. The amounts of each can be therapeutically effective alone - in which case the additive effects can overcome cancers resistant to treatment by monotherapy. The amounts of any can also be subtherapeutic - to minimize adverse effects, particularly in sensitive patients.

It is understood that the treatment of cancer depends on the type of cancer. For example, lung cancer is treated differently as a first line therapy than are colon cancer or breast cancer treated. Even within lung cancer, for example, first line therapy is different from second line therapy, which in turn is different from third line therapy. Newly diagnosed patients might be treated with cisplatinum containing regimens. Were that to fail, they move onto a second line therapy such as a taxane. Finally, if that failed, they might get a tyrosine kinase EGFR inhibitor as a third line therapy. Further, the regulatory approval process differs from country to country. Accordingly, the accepted treatment regimens can differ from country to country. Nevertheless, the compounds of the present invention, or pharmaceutically acceptable salts or *N*-oxides thereof, can be beneficially co-administered in conjunction or combination with other such cancer therapeutic compounds. Such other compounds include, for example, a variety of cytotoxic agents (alkylators, DNA topoisomerase inhibitors, antimetabolites, tubulin binders); inhibitors of angiogenesis; and different other forms of therapies including kinase inhibitors such as Tarceva^{™}, monoclonal antibodies, and cancer vaccines. Other such compounds that can be beneficially co-administered with the compounds of the present invention include doxorubicin, vincristine, cisplatin, carboplatin, gemcitabine, and the taxanes. Thus, the compositions of the present invention include a compound according to Formula (I), or a pharmaceutically acceptable salt or N-oxide thereof, and an anti-neoplastic, anti-tumor, anti-angiogenic, or chemotherapeutic agent.

The compounds of the present invention, or pharmaceutically acceptable salts or N-oxides thereof, can also be effectively administered in conjunction with other therapeutic compounds, aside from cancer therapy. For example, therapeutic agents effective to ameliorate adverse side-effects can be advantageous co-agents with the compounds of the present invention.

### I. Assay for inhibition of c-Kit in intact cells

The ability of compounds to inhibit the tyrosine kinase activity of c-Kit was determined in a cell-based ELISA assay using the H526 cell line (ATCC # CRL-5811), which was originally derived from a human small cell lung cancer. The assay determines the ability of compounds to block ligand-stimulated tyrosine phosphorylation of the wild-type c-Kit receptor protein that is endogenously expressed in H526 cells. Cells are pre-incubated with compounds at various concentrations prior to addition of stem cell factor (SCF), the ligand for the c-Kit receptor tyrosine kinase. Cell lysates are then prepared and the c-Kit protein is captured onto a c-Kit antibody-coated 96-well ELISA plate. The phosphotyrosine content of the receptor protein is then monitored by quantitation of the degree of binding of an antibody that recognizes only the phosphorylated tyrosine residues within the captured protein. The antibody used has a reporter enzyme (e.g. horseradish peroxidase, HRP) covalently attached, such that binding of antibody to phosphorylated c-Kit can be determined quantitatively by incubation with an appropriate HRP substate.

The stock reagents used are as follows:
**Cell lysis buffer:**
   50 mM Tris-HCl, pH 7.4
   150 mM NaCl
   10% Glycerol
   1% Triton X-100
   0.5 mM EDTA
   1 µg/mL leupeptin
   1 µg/mL aprotinin
   1 mM Sodium orthovanadate
**Anti c-Kit antibody:**
   0.5µg/mL anti c-Kit Ab-3 (Lab Vision, catalog #MS289P1) in 50mM Sodium bicarbonate, pH 9.
**ELISA Assay plates:**
   ELISA assay plates are prepared by addition of 100µL of anti c-Kit antibody to each well of a 96-well Microlite-2 plate (Dynex, catalog # 7417), followed by incubation at 37°C for 2h. The wells are then washed twice with 300µL wash buffer.
**Plate wash buffer:**
   PBS containing 0.5% Tween-20 (PBST)
**Cell assay medium:**
   RPMI with 0.1 % BSA
**pY20-HRP:**
   25ng/mL pY20-HRP (Calbiochem, catalog # 525320) in PBS, containing 0.5% Tween-20, 5% BSA, 1 mM Sodium orthovanadate
**HRP substrate:**
   Chemoluminescent detection reagent (Pierce, catalog # 37075)
**Assay protocol:**
   Cultures of H526 cells, growling in RPMI with 10% fetal calf serum, were collected by centrifugation, washed twice with PBS, and suspended in cell assay medium. Cells were then distributed into a V-bottom 96-well plate at 7.5 x 10⁴ cells per well in 100µL cell assay medium.

Compound dilutions were prepared from 10mM DMSO stocks by dilution in cell assay medium, the final concentration of DMSO in the assay being 0.1%. To compound incubation wells, 50µL of the test compound was added (compounds are assayed at concentrations between 0.1nM and 100µM); to positive and negative control wells, 50µL cell assay medium containing 0.1% DMSO was added. The cells were then incubated with compound at 37°C for 3h. SCF (R&D Systems, catalog #255-SC-010) was then added in order to stimulate the Kit receptor and induce its tyrosine phosphorylation. Then, 10µL of a 1.6µg/mL solution of SCF in cell assay medium was added to all wells apart from the negative control wells, and the cells were incubated for an additional 15min at 37°C. Following the addition of ice-cold PBS, the plate was centrifuged at 1000rpm for 5min, the medium removed by aspiration, and the cell pellet lysed by the addition of 120µL ice-cold cell lysis buffer per well. The plate was kept on ice for 20min and 100µL of the cell lysates from each well were then transferred to the wells of an ELISA assay plate and incubated at 4°C for 16h.

Following incubation of the cell lysates in the ELISA plate, the wells were washed 4 times with 300µL wash buffer, then 100µL of the phosphotyrosine detection antibody pY20-HRP was added to each well and the plate incubated at rt for 2h. The wells were then washed 4 times with 300µL wash buffer. Then, 50µL of the chemiluminescent HRP substrate was added to each well for luminometric quantitation of the amount of antiphosphotyrosine-HRP conjugate bound to the plate.

Comparison of the assay signals obtained in the presence of compound with those of the positive and negative controls (cells incubated in the presence or absence of SCF, with no compound added), allows the degree of inhibition of c-Kit receptor tyrosine phosphorylation to be determined over a range of compound concentrations. These inhibition values were fitted to a sigmoidal dose-response inhibition curve to determine the IC₅₀ values (i.e. the concentration of compound that inhibits SCF-induced tyrosine phosphorylation of the c-Kit protein by 50%).

The **EXAMPLES** of this invention reduced the level of SCF-induced tyrosine phosphorylation of Kit in intact H526 cells as determined in the above assay with IC₅₀ values between 15µM and 0.1nM.

### EXPERIMENTAL

The **EXAMPLES** of the present invention were prepared according to the following procedures by the methods illustrated in the following schemes. Appropriate solvents, temperatures, pressures and other reaction conditions may be readily selected by one of ordinary skill in the art. Similarly, suitable starting materials may be commercially obtained or readily prepared.

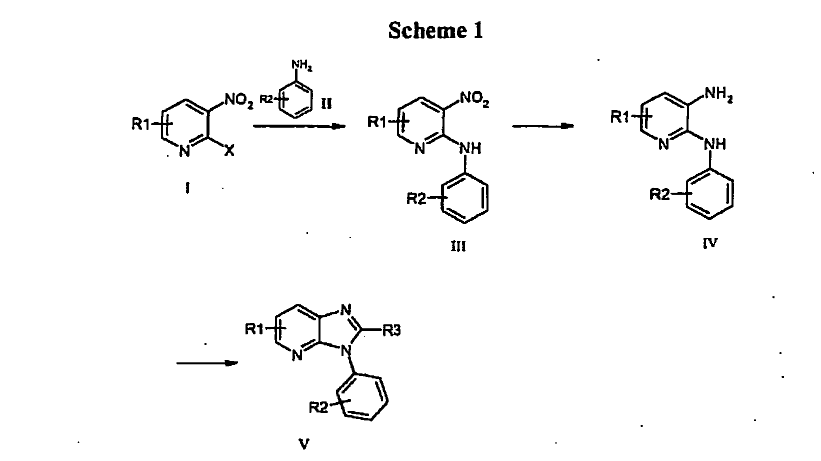

In **Scheme 1,** diarylamines **(III)** may be produced from the condensation of nitropyridines (I, X = F, Cl, Br, OMs, OTs) with substituted anilines **(II).** Coupling of the anilines **(II)** may also be achieved where X = I, Br, Cl, OTf by utilisation of Pd(0) mediated Buchwald-Hartwig-type conditions (such as those described in the Journal of Organic Chemistry (1996), 61(21), 7240) or with Cu(I) catalysis. Reduction of **(III)** to give the diaminopyridines **(IV)** may be achieved using, for example, hydrogen in the presence of a suitable transition metal catalyst (palladium, platinum, ruthenium, nickel), iron, zinc or tin under acidic conditions, with sodium hydrosulphite or with tin(II)chloride dihydrate. Cyclisation of **(IV)** to the benzimidazoles **(V)** may be achieved by reaction with a corresponding carboxylic acid, acid halide, acid anhydride or an orthoformate (e.g. (MeO)₃CH) and an acid such as formic or *p*-toluenesulphonic acid. Under certain conditions used to reduce **(III)** e.g. iron powder in formic acid, conversion to the imidazo[4,5-*b*]pyridines **(V)** may be achieved in one pot. Also, by inclusion of trimethyl orthoformate into a hydrogenation mixture with **(III),** allows the direct conversion to (V).

**Scheme 2** below shows that N-aryl-3*H*-imidazo[4,5-*b*]pyridines **(V)** (and their 1*H*-isomers **Va)** may also be formed via the process whereby imidazo[4,5-*b*]pyridines **(VIII)** may be arylated under Pd(0) mediated conditions similar to those disclosed in Journal of the American Chemical Society (2000), 122, 7600. Separation of the isomers **(V)** and **(Va)** may be achieved by a number of means known to those skilled in the art including, but not limited to, chromatographic means or through crystallisation from a suitable solvent. Imidazo[4,5-*b*]pyridines **(VIII)** may be produced from the cyclisation of the anilides **(VII)** with acids such as, but not limited to, acetic, p-toluenesulphonic, hydrochloric, sulphuric or phosphoric acid. In turn the anilides **(VII)** can be prepared by reaction of diaminopyridines with acid halides or anhydrides or with carboxylic acids in the presence of appropriate coupling reagents known to those skilled in the art such as, but not limited to, EDC, DCC, HOAt, HOBt, HATU, TBTU, CDI including solid supported versions of these solution phase reagents. Where R3 = H compounds such as **(VII)** may be prepared by formylation of **(VI)** with alkyl formates (e.g. methyl formate). In the processes described above, conversion of **(VI)** into **(VII)** may also lead to the partial or complete conversion to **(VIII)** e.g. when R3 = H by heating in the presence of formic acid.

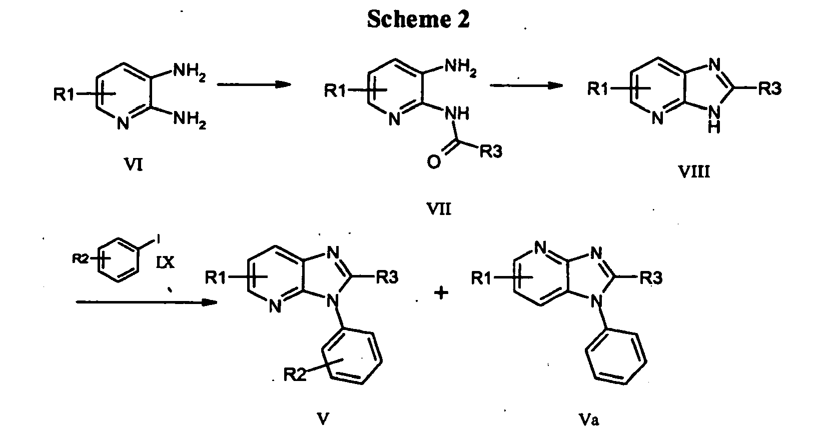

Functionalities R1 and R2, may for the most part be included into the target molecules through appropriate choice of starting materials e.g. of type **(I), (II), (VI)** and **(IX).** Where the final functionality is not available directly through this process, or where such functionality may be compromised during the subsequent chemistry to build the final molecule, then alternative functionalities may be used and subsequently transformed into the final desired functionality by methods, and at points in the sequence, readily determined by one skilled in the art. For example, a non-exhaustive list of such transformations includes the conversions: OMe→OH (BBr₃), NH₂→Cl (NaNO₂, CuCl), Br→CN (Pd₂(dba)₃, Zn(CN)₂, DPPF), Me→CO₂H (KMnO₄), CO₂H→CO₂Me (MeOH, H₂SO₄), OH→OAlkyl (Alkyl halide, base), CO₂H→CONR'R" (EDC, HOAt, DIPEA, HNR'R"), Br→CO₂Me (Pd₂(dba)₃, DPPF, CO(g), MeOH), Br→CO₂H (^{t}BuLi, CO₂), Ar-H→Ar-Br (NBS), CN→CO₂H (conc. H₂SO₄), Br→NR'R" (Pd₂(dba)₃, DPPF, HNR'R"). More specific and representative examples of the incorporation of such functionality into target molecules are shown below in **Schemes 3-5.**

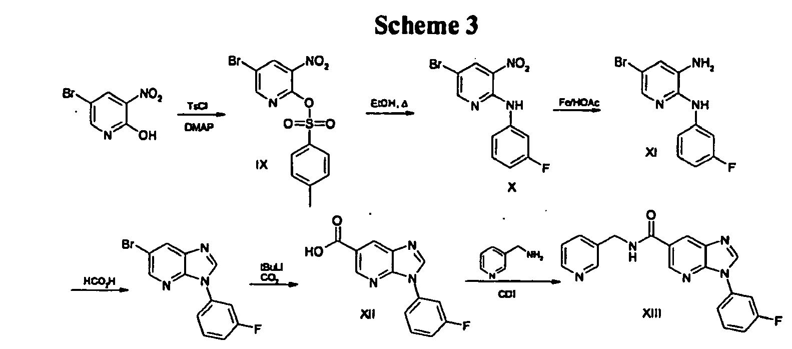

5-Bromo-3-nitro-2-pyridol may be reacted with p-toluenesulphonyl chloride in the presence of DMAP to give the tosylate **(IX),** which can be condensed with 3-fluoroaniline to give the secondary aniline **(X).** Reduction of this intermediate may be achieved with iron powder in acetic acid and the product **(XI)** then cyclised to the imidazo[4,5-b]pyridine with formic acid. Treatment of this material with *tert-*butyllithium followed by quenching of the intermediate anion with carbon dioxide and acid gives the carboxylic acid derivative **(XII).** This may be coupled with 3-aminomethylpyridine using 1,1'-carbonyldiimidazole to give amide **(XIII).**

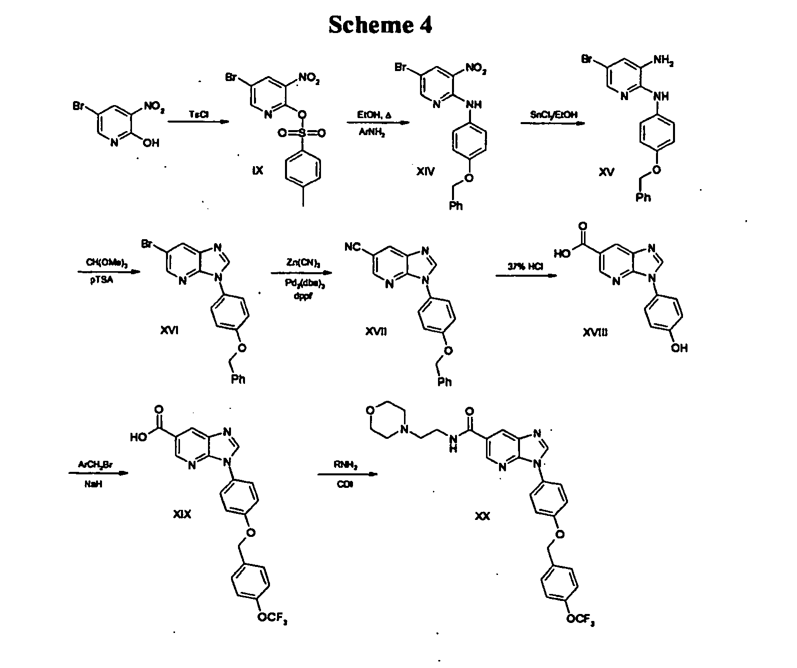

Similarly, in **Scheme 4** the tosylate **(IX)** can be condensed with 4-benzyloxyaniline in ethanol to give nitropyridine **(XIV).** This can be reduced with tin (II) chloride dihydrate in refluxing ethanol to give the diamine **(XV),** which may be cyclised to the imidazo[4,5-b]pyridine **(XVI)** with trimethyl orthoformate in the presence of p-toluenesulphonic acid. As an alternative to the metalation shown in **Scheme 3,** a cyano group may be introduced through Pd(0) mediated means using zinc cyanide to give **(XVII),** although Rosenmund-von Braun (CuCN) conditions are successful also. Acid hydrolysis of the nitrile also facilitates debenzylation to give **(XVIII),** which may be alkylated using, for example, a benzyl bromide derivative in the presence of a base such as sodium hydride, to give on hydrolytic work-up, the ether **(XIX).** 1,1'-Carbonyldiimidazole mediated coupling with 2-morpholin-4-ylethylamine provides target compound **(XX).**

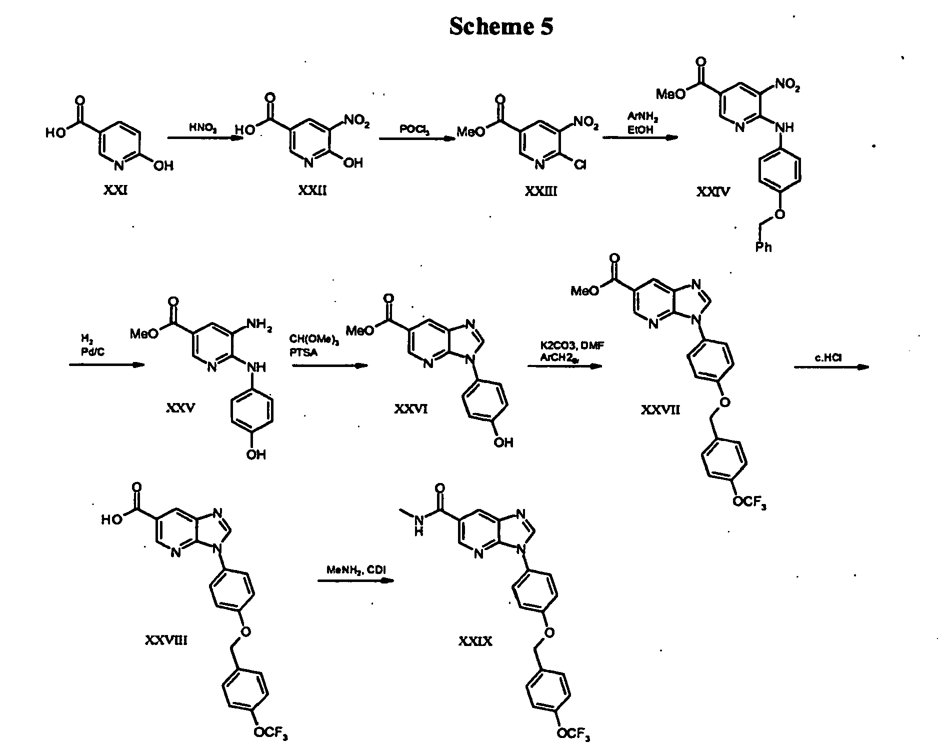

Another alternative route employed in the synthesis of derivatised imidazo[4,5-b]pyridines is shown in Scheme 5. 6-Hydroxynicotinic acid **(XXI)** may be nitrated in the presence of red fuming nitric acid and the product **(XXII)** chlorinated using PCl₅/POCl₃ followed by careful MeOH quenching to give intermediate **(XXIII).** This may be reacted with an aniline, for example, 4-benzyloxyaniline as described previously to give the nitro-anilino-pyridines such as **(XXIV),** which can be reduced (e.g. by catalytic hydrogenation) and cyclised (e.g. trimethyl orthoformate and PTSA) as before to give phenol **(XXVI).** This phenol may be alkylated, for example with a benzyl bromide derivative in the presence of potassium carbonate, and the ester group hydrolysed under acidic conditions and coupled with an amine such as methylamine using CDI to give target amide **(XXIX).**

Definitions: EDC = ethyl dimethylaminopropylcarbodiimide hydrochloride, HOAt = 1-hydroxyazabenzotriazole, HOBt = 1-hydroxybenzotriazole, CDI = 1,1'-carbonyldiimidazole, TBTU = O-benzotriazole-N,N,N',N'-tetramethyluronium tetrafluoroborate, HATU = azabenzotriazolyl-N,N,N'-,N'-tetramethyluronium hexafluorophosphate, DIPEA = diisopropylethylamine, TEA = triethylamine, DMF = N,N-dimethylformamide, NMP = N-methylpyrrolidinone, DCM = dichloromethane, DMAP = 4-dimethylaminopyridine, TFA = trifluoroacetic acid, Boc = ^{t}butoxycarbonyl, Fmoc = fluorenylmethyloxycarbonyl, DMSO = dimethylsulphoxide, OMs = OSO₂Me, OTs = OSO₂-(4-Me)Ph, OTf= OSO₂CF₃, DPPF/dppf = 1,1'-bis(diphenylphosphino)ferrocene, dba = dibenzylideneacetone, NBS = N-bromosuccimimide, HCl(aq) = aqueous hydrochloric acid, DMA = N,N-dimethylacetamide, MeOH = methanol, EtOH = ethanol, EtOAc = ethyl acetate, THF = tetrahydrofuran, HOAc = acetic acid, DMF = N,N-dimethylformamide, HPLC = high performance liquid chromatography,

### EXAMPLE R1

3-{4-[(4-(Trifluoromethoxy)benzyl)oxy]phenyl}-*N*-(2-morpholin-4-ylethyl)-3*H-*imidazo[4,5-*b*]pyridine-6-carboxamide

**a)** 5-Bromo-3-nitropyridin-2-ol (30.00g, 0.137mmol) and *p*-toluenesulfonyl chloride (30.03g, 0.158mmol) were suspended in CH₂Cl₂ under N₂ and treated dropwise with triethylamine (38.2mL, 0.274mmol) prior to the addition of DMAP (3.35g, 0.027mmol). After stirring at rt for 16h the mixture was diluted with CH₂Cl₂ (500mL) then washed with 1M HCl(aq) (2 x 500mL) and brine (500mL). The aqueous layer was back-extracted with CH₂Cl₂ (200mL) and the combined organic layers dried over Na₂SO₄, and concentrated *in vacuo*. The crude material thus isolated was chromatographed over silica gel eluting with 50% EtOAc/hexane to give, on evaporation, a solid which was crystallized from 50% EtOAc/hexane to afford 5-bromo-3-nitropyridin-2-yl 4-methylbenzenesulfonate. ¹H NMR (400MHz, CDCl₃) δ 2.51 (s, 3H), 7.43 (dd, 2H, *J=* 8.4, 0.4 Hz), 7.99 (dt, 2H, *J* = 6.8, 2.0 Hz), 8.52 (d, 1H, *J* = 2.4 Hz), 8.60 (d, 1H, *J* = 2.4 Hz).

**b)** 5-Bromo-3-nitropyridin-2-yl 4-methylbenzenesulfonate (38.2g, 0.102mmol) and 4-benzyloxyaniline (24.13g, 0.102mmol) were suspended in 900mL of toluene under N₂, treated with triethylamine (14.27mL, 0.102mmol) and the mixture heated at 110°C for 16h. Then, the reaction mixture was diluted with CH₂Cl₂ (1L), washed with 2M HCl(aq) (4 x 500mL) and brine (500mL), and the combined organic extracts dried over Na₂SO₄ and then concentrated *in vacuo*. The crude product thus isolated was crystallized twice from ethanol to yield *N*-[4-(benzyloxy)phenyl]-5-bromo-3-nitropyridin-2-amine. ¹H NMR (400MHz, CDCl₃) δ 5.11 (s, 2H), 7.04 (d, 2H, *J*= 9.2 Hz), 7.34-7.50 (m, 7H), 8.48 (d, 1H, *J*= 2.4 Hz), 8.66 (d, 1H, *J*= 2.4 Hz), 9.94 (bs, 1H).

**c)** *N*-[4-(Benzyloxy)phenyl]-5-bromo-3-nitropyridin-2-amine (28.5g, 0.071mmol) and tin (II) chloride dihydrate (160.67g, 0.712mmol) were dissolved in 500mL ethanol under N₂ and the reaction mixture heated at reflux (70°C) for 30min. Sodium bicarbonate was then added to the cooled mixture until the pH was greater than 9.5, and then the mixture was filtered through celite which was washed with methanol. The combined organic phases were concentrated *in vacuo* before being re-constituted in CH₂Cl₂, drying over Na₂SO₄, and concentration *in vacuo* to yield *N*²-[4-(benzyloxy)phenyl]-5-bromopyridine-2,3-diamine. ¹H NMR (400MHz, CDCl₃) δ 3.44 (bs, 2H), 5.07 (s, 2H), 6.02 (bs, 1 H), 6.97 (dt, 2H, *J* = 5.6, 3.6 Hz), 7.12 (d, 1H, *J* = 2.0 Hz), 7.32 (dt, 2H, *J*= 4.8, 0.4 Hz), 7.34-7.48 (m, 5H), 7.84 (d, 1H, *J*= 2 Hz); MS (ES+): *m*/*z* 371[MH⁺], 372 (35) [MH²⁺]

**d)** Trimethyl orthoformate (9.0mL, 81mmol) and *p*-toluenesulphonic acid (384mg, 2.0mmol) were added to a solution of *N*²-[4-(benzyloxy)phenyl]-5-bromopyridine-2,3-diamine (3g, 8.1mmol) in dichloromethane (40mL) and the reaction mixture stirred at rt for 4h. Next, the precipitated material was collected by filtration, redissolved in CH₂Cl₂ and the solution washed with 10% NaOH(aq) (200mL) and brine (200mL), then dried over MgSO₄ and concentrated *in vacuo* to afford 3-[4-(benzyloxy)phenyl]-6-bromo-3*H*-imidazo[4,5-*b*]pyridine. ¹H NMR (400MHz, CDCl₃) δ 5.14 (s, 2H), 7.16 (d, *J*= 8.8Hz, 2H), 7.33-7.47 (m, 5H), 7.58 (d, *J* = 8.8Hz, 2H), 8.25 (s, 1H), 8.28 (d, *J*= 2Hz, 2H) and 8.46 (d, *J*= 2Hz, 2H); MS (ES+): *m*/*z* 380 [Br⁷⁹MH⁺], 382 [Br⁸¹MH⁺].

**e)** 3-[4-(Benzyloxy)phenyl]-6-bromo-3*H*-imidazo[4,5-*b*]pyridine (2g, 5.26mmol), Zn(CN)₂(371mg, 3.16mmol), Pd₂(dba)3(101mg, 0.11mmol) and dppf (122mg, 0.22mmol) in degassed DMF/H₂O (100:1) (15mL) was stirred at 120°C under N₂ for 20h. The mixture was then cooled to rt and treated with NH₄Cl:NH₄OH:H₂O (4:1:4) (45mL) and reheated to 80°C for 30min and then stirred at 0°C for another 30min. The precipitated solids were isolated by filtration and purified by chromatography over silica gel eluting with 50% EtOAc/hexane to give 3-[4-(benzyloxy)phenyl]-3*H*-imidazo[4,5-*b*]pyridine-6-carbonitrile. ¹H NMR (400MHz, D6-Acetone) δ 5.16 (s, 2H), 7.18 (d, *J* = 8Hz, 2H), 7.36-7.47 (m, 5H), 7.57 (d, *J*= 8Hz, 2H), 8.41 (s, 1H), 8.44 (d, *J=* 2Hz, 1H) and 8.70 (d, *J*= 2Hz, 1H); MS (ES+): *mlz* 327 [MH⁺].

**f)** 3-[4-(Benzyloxy)phenyl]-3*H*-imidazo[4,5-*b*]pyridine-6-carbonitrile (650mg) in 37% HCl (20mL) was stirred at 100°C for 48h. The reaction mixture was then cooled to rt and crude 3-(4-hydroxyphenyl)-3*H*-imidazo[4,5-*b*]pyridine-6-carboxylic acid was collected by filtration. ¹H NMR (400MHz, D6-DMSO) δ 6.91 (d, *J* = 8Hz, 2H), 7.58 (d, *J* = 8Hz, 2H), 8.54 (d, *J* = 2Hz, 1H), 8.86 (s, 1H) and 8.9 (d, *J* = 2Hz, 1H); MS (ES+): *m*/*z* 256 [MH⁺].

**g)** A mixtue of 3-(4-hydroxyphenyl)-3*H*-imidazo[4,5-*b*]pyridine-6-carboxylic acid (54mg, 0.21mmol), tetrabutylammonium iodide (8mg, 0.021mmol), 4-(trifluoromethoxy)benzyl bromide (0.13mL, 0.84mmol) and sodium hydride (50.4mg, 1.26mmol) in DMF (3mL) was stirred at rt under nitrogen for 4h. Then, water (3mL) was added and the mixture washed with EtOAc (10mL). The aqueous phase was acidified with 6N HCl(aq) and the resulting precipitate isolated by filtration. The EtOAc wash was concentrated *in vacuo* and the residue dissolved in MeOH and stirred with 6N NaOH(aq) for 2h prior to acidification (2M HCl(aq) and filtration of product. The combined solids were dried *in vacuo* to give 3-{4-[(4-(trifluoromethoxy) benzyl)oxy]phenyl}-3*H*-imidazo[4,5-*b*]pyridine-6-carboxylic acid. ¹H NMR (400MHz, D6-DMSO) δ 5.26 (s, 2H), 7.26 (d, *J* = 8.9Hz, 2H), 7.42 (d, *J* = 8.2Hz, 2H), 7.63 (d, *J* = 8.2Hz, 2H), 7.82 (d, *J* = 8.9Hz, 2H), 8.61 (d, *J* = 1.6Hz, 1H), 8.95 (s, 1H), 8.97 (d, *J* = 1.6Hz, 1H) and 13.2 (br.s, 1H); MS (ES+): *mlz* 430 [MH⁺].

**h)** A mixture of 3-{4-[(4-(trifluoromethoxy)benzyl)oxy]phenyl}-3*H-*imidazo[4,5-*b*]pyridine-6-carboxylic acid (42mg, 0.1mmol), CDI (32.4mg, 0.2mmol) and DIPEA (87µL, 0.5mmol) in dry THF (2mL) was stirred at 60°C for 2h and then treated with 2-(morpholin-4-yl)ethylamine (26µL, 0.2mmol). After a further 2h at 60°C the mixture was concentrated *in vacuo* and the residue chromatographed over silica gel eluting with 5%MeOH/CHCl₃ to give 3-{4-[(4-(trifluoromethoxy)benzyl)oxy]phenyl}-*N*-(2-morpholin-4-ylethyl)-3*H*-imidazo[4,5-*b*]pyridine-6-carboxamide. ¹H NMR (400MHz, D4-MeOH) δ 2.57 (br.s, 4H), 2.65 (t, *J* = 8Hz, 2H), 3.60 (t, *J*= 8Hz, 2H), 3.71 (t, *J*= 8Hz, 4H), 5.21 (s, 2H), 7.22 (dd, *J*= 6.8 and 2Hz, 2H), 7.30 (d, *J*= 8.4Hz, 2H), 7.59 (d, *J*= 8.8Hz, 2H), 7.71 (dd, *J*= 6.8 and 2Hz, 2H), 8.57 (d, *J*= 1.6Hz, 1H), 8.71 (s, 1H) and 8.90 (d, *J*= 1.6Hz, 1H); MS (ES+): *m*/*z* 542 [MH⁺].

The following examples were prepared according to the procedure described above for **EXAMPLE R1,** utilising the appropriate aniline and amine in place of 4-benzyloxyaniline (step a)) and 2-(morpholin-4-yl)ethylamine (step h)), respectively.

### EXAMPLE R2

3-{4-[(4-(Trifluoromethoxy)benzyl)oxy]phenyl}-*N*-(2-methoxyethyl)-3*H-*imidazo[4,5-*b*]pyridine-6-carboxamide. MS (ES+): *m*/*z* 487 [MH⁺]

### EXAMPLE R3

3-{3-[(4-(Trifluoromethoxy)benzyl)oxy]phenyl}-*N*-(pyridine-3-ylmethyl)-3*H-*imidazo[4,5-*b*]pyridine-6-carboxamide. MS (ES+): *m*/*z* 520 [MH⁺]

### EXAMPLE R4

3-{3-[(4-(Trifluoromethoxy)benzyl)oxy]phenyl}-*N*-(2-methoxyethyl)-3*H-*imidazo[4,5-*b*]pyridine-6-carboxamide. MS (ES+): *m*/*z* 487 [MH⁺]

### EXAMPLE R5

3-{4-[(4-(Trifluoromethoxy)benzyl)oxy]phenyl}-*N*-(3-(4-methylpiperazin-1-yl)propyl)-3*H*-imidazo[4,5-*b*]pyridine-6-carboxamide. MS (ES+): *m*/*z* 569 [MH⁺]

### EXAMPLE R6

3-{4-[(4-(Trifluoromethoxy)benzyl)oxy]phenyl}-*N*-(2-dimethylaminoethyl)-3*H-*imidazo[4,5-*b*]pyridine-6-carboxamide. MS (ES+): *m*/*z* 500 [MH⁺]

### EXAMPLE R7

3-{4-[(4-(Trifluoromethoxy)benzyl)oxy]phenyl}-*N*-(2-piperidin-1-ylethyl)-3*H-*imidazo[4,5-*b*]pyridine-6-carboxamide. MS (ES+): *m*/*z* 540 [MH⁺]

### EXAMPLE R8

3-{4-[(4-(Trifluoromethoxy)benzyl)oxy]phenyl}-*N*-(pyridine-3-ylmethyl)-3*H-*imidazo[4,5-*b*]pyridine-6-carboxamide. MS (ES+): *m*/*z* 520 [MH⁺]

### EXAMPLE R9

3-{4-[(4-(Trifluoromethoxy)benzyl)oxy]phenyl}-*N*-methyl-3*H*-imidazo[4,5-*b*]pyridine-6-carboxamide. MS (ES+): *m*/*z* 443 [MH⁺]

### EXAMPLE R10

3-{4-[(4-(Trifluoromethoxy)benzyl)oxy]phenyl}-*N*-ethyl-3*H*-imidazo[4,5-*b*]pyridine-6-carboxamide. MS (ES+): *m*/*z* 457 [MH⁺]

### EXAMPLE R11

3-{4-[(4-(Trifluoromethoxy)benzyl)oxy]phenyl}-*N*-(2-pyrrolidin-1-ylethyl)-3*H-*imidazo[4,5-*b*]pyridine-6-carboxamide. MS (ES+): *m*/*z* 526 [MH⁺]

### EXAMPLE R12

3-{3-[(4-(Trifluoromethoxy)benzyl)oxy]phenyl}-*N*-methyl-3*H*-imidazo[4,5-b]pyridine-6-carboxamide. MS (ES+): m/z 443 [MH⁺]

### EXAMPLE R13

3-{3-[(4-(Trifluoromethoxy)benzyl)oxy]phenyl}-*N*-ethyl-3*H*-imidazo[4,5-b]pyridine-6-carboxamide. MS (ES+): m/z 457 [MH⁺]

### EXAMPLE R14

3-{3-[(4-(Trifluoromethoxy)benzyl)oxy]phenyl}-*N*-(3-methoxypropyl)-3*H-*imidazo[4,5-b]pyridine-6-carboxamide. MS (ES+): m/z 501 [MH⁺]

### EXAMPLE R15

3-{3-[(4-(Trifluoromethoxy)benzyl)oxy]phenyl}-*N*-(2-dimethylaminoethyl)-3*H-*imidazo[4,5-*b*]pyridine-6-carboxamide. MS (ES+): *m*/*z* 500 [MH⁺]

### EXAMPLE R16

3-(4-Methoxyphenyl)-*N*-(pyridin-3-ylmethyl)-3*H*-imidazo[4,5-*b*]pyridine-6-carboxamide. ¹H NMR (400 MHz, CD₃OD/CDCl₃): δ 3.87 (3H, s), 4.64 (2H), 7.10 (2H, d, J = 9.0 Hz), 7.36 (1H, dd, J = 5.0 Hz, 7.9 Hz), 7.61 (2H, d, J = 9.0 Hz), 7.83 (1H, dt, J = 1.6 Hz, 7.8 Hz), 8.41 (1H, dd, J = 1.3 Hz, 4.8 Hz), 8.49 (1H, s), 8.55 (1H, br), 8.59 (1H, d, J = 2.0 Hz), 8.95 (1H, d, J = 2.0 Hz); MS (ES+): *m*/*z* 360 [MH⁺]

### EXAMPLE R17

3-(4-Methoxyphenyl)-*N*-(2-morpholin-4-ylethyl)-3*H*-imidazo[4,5-*b*]pyridine-6-carboxamide. ¹H NMR (400 MHz, CDCl₃): δ 2.54 (4H, br), 2.65 (2H, t, J = 6.0 Hz), 3.62 (2H, quart, J = 5.5 Hz), 3.89 (3H, s), 6.94 (1H, br), 7.10 (2H, d, J = 8.9 Hz), 7.61 (2H, d, J = 8.9 Hz), 8.35 (1H, s), 8.53 (1H, d, J = 1.9 Hz), 8.91 (1H, J = 1.9 Hz); MS (ES+): *m*/*z* 382 [MH⁺]

### EXAMPLE R18

3-(4-Methoxyphenyl)-*N*-(pyridin-2-ylmethyl)-3*H*-imidazo[4,5-*b*]pyridine-6-carboxamide. ¹H NMR (400 MHz, CDCl₃): δ 3.90 (3H, s), 4.83 (2H, d, J = 4.7 Hz), 7.11 (2H, d, J = 8.9 Hz), 7.24-7.28 (1H, m), 7.38 (1H, d, J = 7.8 Hz), 7.63 (2H, d, J = 8.9 Hz), 7.73 (1H, td, J = 1.6 Hz, 7.7 Hz), 7.98 (1H, br), 8.37 (1H, s), 8.58 (1H, d, J = 4.7 Hz), 8.67 (1H, d, J = 1.9 Hz), 9.04 (1H, d, J = 1.9 Hz); MS (ES+): *m*/*z* 360 [MH⁺]

### EXAMPLE R19

3-(4-Methoxyphenyl)-*N*-(3-methoxypropyl)-3*H*-imidazo[4,5-*b*]pyridine-6-carboxamide. ¹H NMR (400 MHz, CDCl₃): δ 1.91 (2H, quint, J = 5.5 Hz), 3.41 (3H,s), 3.61 (2H, t, J = 5.5 Hz), 3.66 (2H, t, J = 5.4 Hz), 3.89 (3H, s), 7.10 (2H, d, J = 8.9 Hz), 7.21 (br), 7.61 (2H, d, J = 8.9 Hz), 8.34 (1H, s), 8.52 (1H, d, J = 2.0 Hz), 8.90 (1H, d, J = 1.9 Hz); MS (ES+): *m*/*z* 341 [MH⁺]

As an alternative route to the imidazopyridines herein described, the following route was also applied toward the synthesis of key intermediate **(XXVI).**

**6-Hydroxy-5-nitronicotinic acid (XXII):** To a 250mL flask were added 6-hydroxynicotinic acid (20g, Aldrich) and 100mL of red fuming nitric acid. The mixture was slowly heated to 50°C (bath temperature) and stirred at this temperature for 8h. Then the temperature was slowly raised to 80°C and the mixture was stirred for another 7h. The mixture was cooled to rt overnight and the yellow precipitate was collected by filtration, washed with water (10mL) and dried. LC-MS: >95% pure. ¹H NMR (CD₃OD, 400 MHz): δ = 8.45 (d, *J* = 2.5 Hz, 1H), 8.85 (d, *J* = 2.5 Hz, 1H).

**6-Chloro-5-nitronicotinic acid methyl ester (XXIII):** A suspension of 6-hydroxy-5-nitronicotinic acid (1.1g, 6.0mmol) and PCl₅ (3.75g, 18.0mmol) in POCl₃ (5 mL) was stirred at 100°C for 2h. Excessive phosphorus oxychloride was evaporated under reduced pressure. The residue was dissolved in 10mL of anhydrous ether and cooled to 0°C, then 10mL of methanol was added dropwise. 10min later, the ether was evaporated under reduced pressure at rt. The remaining methanol solution was diluted with water (40mL), and the light-yellow solid was collected by filtration. ¹H NMR (CDCl₃, 400 MHz): δ = 4.03 (s, 3H), 8.77(d, *J* = 2.1 Hz, 1H), 9.18 (d, *J* = 2.1 Hz, 1H).

**6-(4-Benzyloxyphenylamino)-5-nitronicotinic acid methyl ester (XXIV):** To a solution of 6-chloro-5-nitronicotinic acid methyl ester (216mg, 1.0mmol) and 4-benzyloxyaniline hydrochloride (280mg, 1.2mmol) in MeOH (10mL) was added ⁱPr₂NEt (0.35mL, 2.0mmol). The resulting mixture was stirred at rt overnight, a red solid precipitated from the mixture, which was collected by filtration. MS (ES, Pos.): *m*/*z* 380 [MH⁺]. ¹H NMR (CDCl₃, 400 MHz): δ = 3.94 (s, 3H), 5.10 (s, 2H), 7.03 (d, *J* = 8.8 Hz, 2H), 7.38-7.46 (m, 5H), 7.50 (d, *J* = 8.8 Hz, 2H), 9.01 (d, *J*= 2.0 Hz, 1H), 9.08 (d, *J* = 2.0 Hz, 1H), 10.2 (br s, 1H).

**3-(4-Hydroxyphenyl)-3*H*-imidazo[4,5-*b*]pyridine-6-carboxylic acid methyl ester (XXVI):** To a suspension of **(XXIV)** (235mg, 0.62mmol) in MeOH (3mL) and ethyl acetate (3mL) was added 10 % Pd-C (47mg) under nitrogen atmosphere. The resulting mixture was hydrogenated at rt overnight. The catalyst was removed by filtration under nitrogen and washed with methanol, the filtrate was concentrated under reduced pressure to give a white solid. MS (ES, Pos.): *m*/*z* 260 [MH⁺]. The hydrogenation product was suspended in trimethyl orthoformate (5mL), and p-toluenesulfonic acid monohydrate (12mg, 0.062mmol) was added. The resulting mixture was stirred at rt overnight. The white solid was collected by filtration. MS (ES, Pos.): *m*/*z* 270 [MH⁺]. ¹H NMR (CD₃OD, 400 MHz): δ = 3.99 (s, 3H), 7.00 (d, *J*= 8.8 Hz, 2H), 7.59 (d, *J*= 8.8 Hz, 2H), 8.71 (d, *J* = 1.8 Hz, 1H), 8.72 (s, 1H), 9.05 (d, *J*= 1.8 Hz, 1H).

## Claims

1. A compound represented by Formula (I) 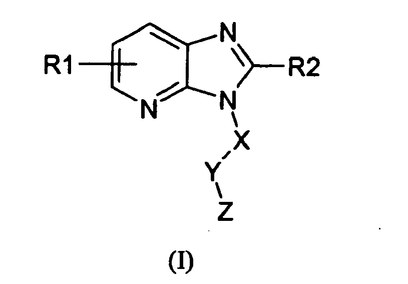 wherein:
R1 is -CO-NR₃R₃₁;
R2 is H, -C₀₋₈alkyl, or -C₃₋₁₀cycloalkyl;
X is a cyclyl or heterocyclyl group optionally substituted with 1 or more substituents chosen from H, halogen, NR₃₂R₃₃, NR₃₂COR₃₃, NR₃₂CO₂R₃₃, NR₃₂SO₂R₃₃ OR₃₂, SR₃₂, SO₂R₃₂, SO₂NR₃₂R₃₃, CO₂R₃₂, CO₂H, CONR₃₂R₃₃, -C₀₋₈alkyl, -C₂₋₈alkenyl, -C₂₋₈alkynyl, CN, CF₃, OCF₃, NO₂, oxo, cyclyl or a heterocyclyl group;
Y is absent, or 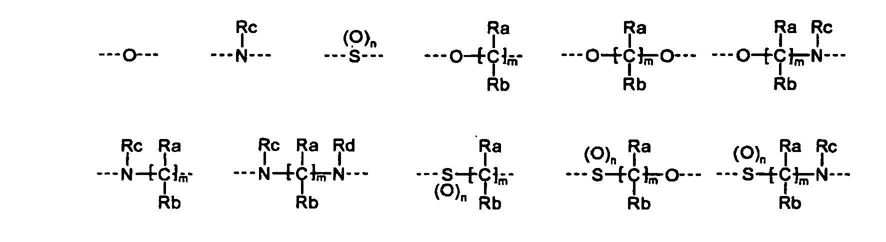 or 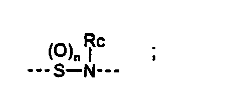 wherein the point of attachment to X can be from either the left or the right of the linkers as shown;
Rₐ and R_{b} each independently is -C₀₋₈alkyl, -C₂₋₈alkenyl, -C₂₋₈alkynyl, -C₃₋₁₀cycloalkyl, -C₃₋₁₀cycloalkenyl, -C₁₋₈alkoxy, -thioC₁₋₈alkyl, carboxyl, -N(C₀₋₈alkyl)(C₀₋₈alkyl), oxo, or hydroxy; or taken together with the C to which they are attached, form a saturated or partially unsaturated 3-10 membered ring optionally containing 0-4 N, O, S, SO, or SO₂ at the ring nodes;
R_{c} and R_{d} each independently is -C₀₋₈alkyl, -C₂₋₈alkenyl, benzyl, or acyl; or taken together, or with Rₐ or R_{b}, form a 3-7 membered saturated or partially unsaturated ring;
m is 0, 1, 2, 3, 4, or 5;
n is 0, 1 or 2;
Z is a cyclyl or heterocyclyl group, optionally substituted with 1 or more substituents chosen from halogen, NR₃₄R₃₅, NR₃₄COR₃₅, NR₃₄CO₂R₃₅, NR₃₄SO₂R₃₅, OR₃₄, SR₃₄, SO₂R₃₄, SO₂NR₃₄R₃₅, CO₂R₃₄, CO₂H, CONR₃₄R₃₅, -C₀₋₈alkyl, -C₂₋₈alkenyl, -C₂₋₈alkynyl, CN, CF₃, NO₂, oxo, cyclyl or a heterocyclyl group; or, when X and Y are present, Z can be -C₁₋₈alkyl or -C₁₋₈alkyl-O-C₁₋₈alkyl; and
R₃, R₃₁, R₃₂, R₃₃, R₃₄ and R₃₅ are independently C₀₋₈alkyl optionally substituted with a heterocyclyl or OH substituent; -C₀₋₈alkyl-C₃₋₈cycloalkyl, CF₃, -C₀₋₈alkyl-O-C₀₋₈alkyl, -C₀₋₈alkyl-N(C₀₋₈alkyl)(C₀₋₈alkyl), -C₀₋₈alkyl-S(O)₀₋₂-C₀₋₈alkyl; or heterocyclyl optionally substituted with -C₀₋₈alkyl, cyclyl or substituted cyclyl substituent;
wherein cyclyl groups represent 3-10 membered aromatic, partially aromatic or non-aromatic cyclyl groups, and heterocyclyl groups respresent 3-10 membered aromatic, partially aromatic or non-aromatic heterocyclyl groups containing one or more atoms chosen independently from N, O and S and their oxides;
or a pharmaceutically acceptable salt or N-oxide thereof.

2. The compound according to claim 1, or a pharmaceutically acceptable salt or N-oxide thereof, wherein X is cyclyl.

3. The compound according to claim 1 or 2, or a pharmaceutically acceptable salt or N-oxide thereof, wherein Y is absent.

4. The compound according to claim 1 or 2, or a pharmaceutically acceptable salt or N-oxide thereof, wherein Y is

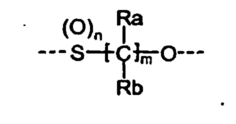

5. The compound according to claim 1 or 2, or a pharmaceutically acceptable salt or N-oxide thereof, wherein Y is

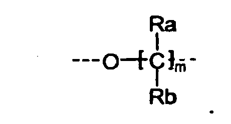

6. The compound according to claim 1 or 2, or a pharmaceutically acceptable salt or N-oxide thereof, wherein Y is

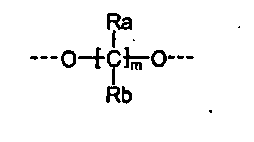

7. The compound according to claim 1 or 2, or a pharmaceutically acceptable salt or N-oxide thereof, wherein Y is

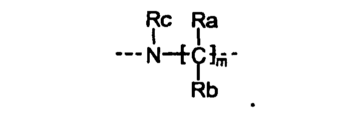

8. A compound consisting of
3-{4-[(4-(trifluoromethoxy)benzyl)oxy]phenyl}-*N*-(2-morpholin-4-ylethyl)-3*H-*imidazo[4,5-*b*]pyridine-6-carboxamide;
3-{4-[(4-(trifluoromethoxy)benzyl)oxy]phenyl}-*N*-(2-methoxyethyl)-3*H-*imidazo[4,5-*b*]pyridine-6-carboxamide;
3-{3-[(4-(trifluoromethoxy)benzyl)oxy]phenyl}-*N*-(pyridine-3-ylmethyl)-3*H-*imidazo[4,5-*b*]pyridine-6-carboxamide;
3-{3-[(4-(trifluoromethoxy)benzyl)oxy]phenyl}-*N*-(2-methoxyethyl)-3*H-*imidazo[4,5-*b*]pyridine-6-carboxamide;
3-{4-[(4-(trifluoromethoxy)benzyl)oxy]phenyl}-*N*-(3-(4-methylpiperazin-1-yl)propyl)-3*H*-imidazo[4,5-*b*]pyridine-6-carboxamide;
3-{4-[(4-(trifluoromethoxy)benzyl)oxy]phenyl}-*N*-(2-dimethylaminoethyl)-3*H-*imidazo[4,5-*b*]pyridine-6-carboxamide;
3-{4-[(4-(trifluoromethoxy)benzyl)oxy]phenyl}-*N*-(2-piperidin-1-ylethyl)-3*H-*imidazo[4,5-*b*]pyridine-6-carboxamide;
3-{4-[(4-(trifluoromethoxy)benzyl)oxy]phenyl}-*N*-(pyridine-3-ylmethyl)-3*H-*imidazo[4,5-*b*]pyridine-6-carboxamide;
3-{4-[(4-(trifluoromethoxy)benzyl)oxy]phenyl}-*N*-methyl-3*H*-imidazo[4,5-*b*]pyridine-6-carboxamide;
3-{4-[(4-(trifluoromethoxy)benzyl)oxy]phenyl}-*N*-ethyl-3*H*-imidazo[4,5-*b*]pyridine-6-carboxamide;
3-{4-[(4-(trifluoromethoxy)benzyl)oxy]phenyl}-*N*-(2-pyrrolidin-1-ylethyl)-3*H-*imidazo[4,5-*b*]pyridine-6-carboxamide;
3-{3-[(4-(trifluoromethoxy)benzyl)oxy]phenyl}-*N*-methyl-3*H*-imidazo[4,5-*b*]pyridine-6-carboxamide;
3-{3-[(4-(trifluoromethoxy)benzyl)oxy]phenyl}-*N*-ethyl-3*H*-imidazo[4,5-*b*]pyridine-6-carboxamide;
3-{3-[(4-(trifluoromethoxy)benzyl)oxy]phenyl}-*N*-(3-methoxypropyl)-3*H-*imidazo[4,5-*b*]pyridine-6-carboxamide;
3-{3-[(4-(trifluoromethoxy)benzyl)oxy]phenyl}-*N*-(2-dimethylaminoethyl)-3*H-*imidazo[4,5-*b*]pyridine-6-carboxamide;
3-(4-methoxyphenyl)-*N*-(pyridin-3-ylmethyl)-3*H*-imidazo[4,5-*b*]pyridine-6-carboxamide;
3-(4-methoxyphenyl)-*N*-(2-morpholin-4-ylethyl)-3*H*-imidazo[4,5-*b*]pyridine-6-carboxamide;
3-(4-methoxyphenyl)-*N*-(pyridin-2-ylmethyl)-3*H*-imidazo[4,5-*b*]pyridine-6-carboxamide;
3-(4-methoxyphenyl)-*N*-(3-methoxypropyl)-3*H*-imidazo[4,5-*b*]pyridine-6-carboxamide;
or a pharmaceutically acceptable salt, or *N*-oxide, thereof.

9. A composition comprising a compound according to any one of claims 1 to 8, or a pharmaceutically acceptable salt or N-oxide thereof, and a pharmaceutically acceptable carrier.

10. A composition comprising a compound according to any one of claims 1 to 8, or a pharmaceutically acceptable salt or N-oxide thereof; and an anti-neoplastic, anti-tumor, anti-angiogenic, or chemotherapeutic agent.

11. A composition comprising a compound according to any one of claims 1 to 8, or a pharmaceutically acceptable salt or N-oxide thereof, and a cytotoxic cancer therapeutic agent.

12. A composition comprising a compound according to any one of claims 1 to 8, or a pharmaceutically acceptable salt or N-oxide thereof, and an angiogenesis inhibiting cancer therapeutic agent.

13. A compound according to any one of claims 1 to 8, or a pharmaceutically acceptable salt or N-oxide thereof, for use in the treatment of a hyperproliferative disorder.

14. A compound according to any one of claims 1 to 8, or a pharmaceutically acceptable salt or N-oxide thereof, for administration with an anti-neoplastic, anti-tumor, anti-angiogenic, or chemotherapeutic agent in the treatment of a hyperproliferative disorder.

15. A compound according to any one of claims 1 to 8, or a pharmaceutically acceptable salt or N-oxide thereof, for use in the treatment of a hyperproliferative disorder selected from breast cancer, head cancer, neck cancer, gastrointestinal cancer, leukemia, ovarian, bronchial, lung, or pancreatic cancer, or small cell lung and colon cancer.

16. A compound according to any one of claims 1 to 8, or a pharmaceutically acceptable salt or N-oxide thereof, for use in the treatment of a hyperproliferative disorder selected from sinonasal natural killer/T-cell lymphoma, testicular cancer (seminoma), thyroid carcinoma, malignant melanoma, adenoid cystic carcinoma, angiosarcoma, anaplastic large cell lymphoma, endometrial carcinoma, and prostate carcinoma.

17. The use of a compound according to any one of claims 1 to 8, or a pharmaceutically acceptable salt or N-oxide thereof, for the manufacture of a medicament for the treatment of a hyperproliferative disorder as defined in any one of claims 13, 15 or 16.

## Patentansprüche

1. Verbindungen der Formel (I) 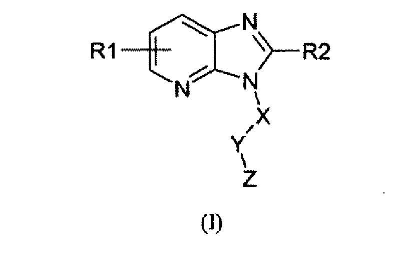 wobei:
R¹ für -CO-NR₃R₃₁ steht;
R² für H, -C₀₋₈-Alkyl oder -C₃₋₁₀-Cycloalkyl steht;
X für eine Cyclyl- oder Heterocyclylgruppe steht, die gegebenenfalls durch einen oder mehrere Substituenten ausgewählt aus H, Halogen, NR₃₂R₃₃, NR₃₂COR₃₃, NR₃₂CO₂R₃₃, NR₃₂SO₂R₃₃, OR₃₂, SR₃₂, SO₂R₃₂, SO₂NR₃₂R₃₃, CO₂R₃₂, CO₂H, CONR₃₂R₃₃, -C₀₋₈-Alkyl, -C₂₋₈-Alkenyl_{,} -C₂₋₈-Alkinyl, CN, CF₃, OCF₃, NO₂, Oxo, einer Cyclyl- oder einer Heterocyclylgruppe substituiert ist;
Y fehlt oder für 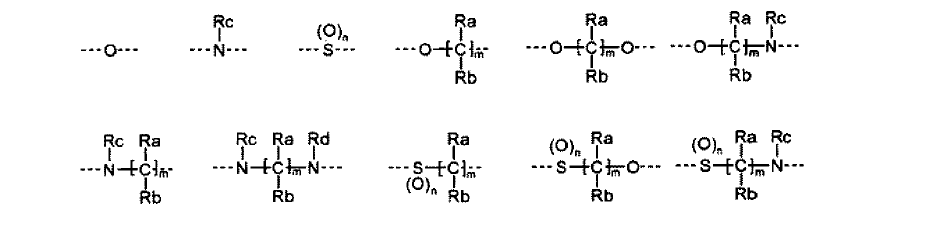 oder 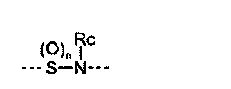 steht;
wobei die Anbindung an X entweder von der linken oder der rechten Seite der Brückenglieder wie gezeigt erfolgen kann;
Rₐ und R_{b} jeweils unabhängig voneinander für -C₀₋₈-Alkyl, -C₂₋₈-Alkenyl, -C₂₋₈-Alkinyl, -C₃₋₁₀-Cycloalkyl, -C₂₋₁₀-Cycloalkenyl, -C₁₋₈-Alkoxy, -Thio-C₁₋₈-alkyl, Carboxyl, - N(C₀₋₈-Alkyl)(C₀₋₈-alkyl), Oxo oder Hydroxy stehen oder zusammen mit dem Kohlenstoff, an den sie gebunden sind, einen gesättigten oder teilweise ungesättigten 3- bis 10-gliedrigen Ring bilden, der gegebenenfalls an den Ringknoten 0-4 N, 0, SO oder SO₂ enthält;
R_{c} und R_{d} jeweils unabhängig voneinander für -C₀₋₈-Alkyl, -C₂₋₈-Alkenyl, Benzyl oder Acyl stehen oder zusammen oder mit Rₐ oder R_{b} einen 3- bis 7-gliedrigen gesättigten oder teilweise ungesättigten Ring bilden;
m für 0, 1, 2, 3, 4 oder 5 steht;
n für 0, 1 oder 2 steht;
Z für eine Cyclyl- oder Heterocyclylgruppe steht, die gegebenenfalls durch einen oder mehrere Substituenten ausgewählt aus Halogen, NR₂₄R₂₅, NR₃₄COR₃₅, NR₃₄CO₂R₃₅, NR₃₄SO₂R₃₅, OR₃₄, SR₃₄, SO₂R₂₄, SO₂NR₃₄R₃₅, CO₂R₃₄, CO₂H, CONR₃₄R₃₅, -C₀₋₈-Alkyl, -C₂₋₈-Alkenyl, -C₂₋₈-Alinyl, CN, CF₃, NO₂, Oxo, einer Cyclyl- oder einer Heterocyclylgruppe substituiert ist oder, wenn X und Y vorhanden sind, Z für -C₁₋₈-Alkyl oder -C₁₋₈-Alkyl-O-C₁₋₈-alkyl steht; und
R₃, R₃₁, R₃₂, R₃₃, R₃₄ und R₃₅ unabhängig voneinander für C₀₋₈-Alkyl, gegebenenfalls substituiert durch einen Heterocyclyl- oder OH-Substituenten, -C₀₋₈-Alkyl-C₂₋₈-cycloalkyl, CF₃, -C₀₋₈-O-C₀₋₈-Alkyl, -C₀₋₈-Alkyl-N(C₀₋₈-alkyl)(C₀₋₈-alkyl), -C₀₋₈-Alkyl-S(O)₀₋₂C₀₋₈-alkyl oder gegebenenfalls durch -C₀₋₈-Alkyl, Cyclyl oder einen substituierten Cyclylsubstituenten substituiertes Heterocyclyl stehen;
wobei Cyclylgruppen für 3- bis 10-gliedrige aromatische, teilweise aromatische oder nichtaromatische Cyclylgruppen stehen und Heterocyclylgruppen für 3- bis 10-gliedrige aromatische, teilweise aromatische oder nichtaromatische Heterocyclylgruppen mit einem oder mehreren Atomen unabhängig voneinander ausgewählt aus N, O und S und ihren Oxiden stehen;
und deren pharmazeutisch unbedenkliche Salze und N-Oxide.

2. Verbindungen nach Anspruch 1 und deren pharmazeutisch unbedenkliche Salze und N-Oxide, in denen X für Cyclyl steht.

3. Verbindungen nach Anspruch 1 oder 2 und deren pharmazeutisch unbedenkliche Salze und N-Oxide, wobei Y fehlt.

4. Verbindungen nach Anspruch 1 oder 2 und deren pharmazeutisch unbedenkliche Salze und N-Oxide, wobei Y für 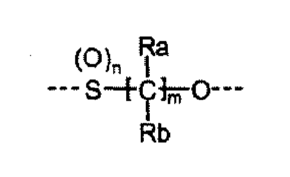 steht.

5. Verbindungen nach Anspruch 1 oder 2 und deren pharmazeutisch unbedenkliche Salze und N-Oxide, wobei Y für 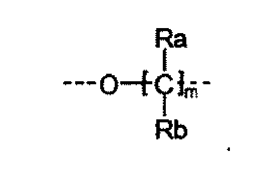 steht.

6. Verbindungen nach Anspruch 1 oder 2 und deren pharmazeutisch unbedenkliche Salze und N-Oxide, wobei Y für 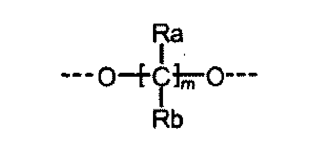 steht.

7. Verbindungen nach Anspruch 1 oder 2 und deren pharmazeutisch unbedenkliche Salze und N-Oxide, wobei Y für 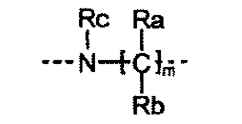 steht.

8. Verbindungen, ausgewählt aus
3-{4-[(4-(Trifluormethoxy)benzyl)oxy]phenyl}-*N*-(2-morpholin-4-ylethyl)-3*H*-imidazo[4,5-*b*]pyridin-6-carbonsäureamid;
3-{4-[(4-(Trifluormethoxy)benzyl)oxy]phenyl}-*N*-(2-methoxyethyl)-3*H*-imidazo[4,5-*b*]pyridin-6-carbonsäureamid;
3-{3-[(4-(Trifluormethoxy)benzyl)oxy]phenyl}-*N-*(pyridin-3-ylmethyl)-3*H*-imidazo[4,5-*b*]pyridin-6-carbonsäureamid;
3-{3-[(4-(Trifluormethoxy)benzyl)oxy]phenyl}-*N*-(2-methoxyethyl)-3*H*-imidazo[4,5-*b*]pyridin-6-carbonsäureamid;
3-{4-[(4-(Trifluormethoxy)benzyl)oxy]phenyl}-*N*-(3-(4-methylpiperazin-1-yl)propyl)-3*H*-imidazo[4,5-*b*]pyridin-6-carbonsäureamid;
3-{4-[(4-(Trifluormethoxy)benzyl)oxy]phenyl}-*N*-(2-dimethylaminoethyl)-3*H*-imidazo[4,5-*b*]pyridin-6-carbonsäureamid;
3-{4-[(4-(Trifluormethoxy)benzyl)oxy]phenyl}-*N*-(2-piperidin-1-ylethyl)-3*H*-imidazo[4,5-*b*]pyridin-6-carbonsäureamid;
3-{4-[(4-(Trifluormethoxy)benzyl)oxy]phenyl}-*N-*(pyridin-3-ylmethyl)-3*H*-imidazo[4,5-*b*]pyridin-6-carbonsäureamid;
3-{4-[(4-(Trifluormethoxy)benzyl)oxy]phenyl}-*N*-methyl-3*H*-imidazo[4,5-*b*]pyridin-6-carbonsäureamid;
3-{4-[(4-(Trifluormethoxy)benzyl)oxy]phenyl}-*N*-ethyl-3*H*-imidazo[4,5-*b*]pyridin-6-carbonsäureamid;
3-{4-[(4-(Trifluormethoxy)benzyl)oxy]phenyl}-*N*-(2-pyrrolidin-1-ylethyl)-3*H*-imidazo[4,5-*b*]pyridin-6-carbonsäureamid;
3-{3-[(4-(Trifluormethoxy)benzyl)oxy]phenyl}-*N*-methyl-3*H*-imidazo[4,5-*b*]pyridin-6-carbonsäureamid;
3-{3-[(4-(Trifluormethoxy)benzyl)oxy]phenyl}-*N*-ethyl-3*H*-imidazo[4,5-*b*]pyridin-6-carbonsäureamid;
3-{3-[(4-(Trifluormethoxy)benzyl)oxy]phenyl}-*N*-(3-methoxypropyl)-3*H*-imidazo[4,5-*b*]pyridin-6-carbonsäureamid;
3-{3-[(4-(Trifluormethoxy)benzyl)oxy]phenyl}-*N*-(2-dimethylaminoethyl)-3*H*-imidazo[4,5-*b*]pyridin-6-carbonsäureamid;
3-(4-Methoxyphenyl)-*N*-(pyridin-3-ylmethyl)-3*H-*imidazo[4,5-*b*]pyridin-6-carbonsäureamid;
3-(4-Methoxyphenyl)-*N*-(2-morpholin-4-ylethyl)-3*H-*imidazo[4,5-*b*]pyridin-6-carbonsäureamid;
3-(4-Methoxyphenyl)-*N*-(pyridin-2-ylmethyl)-3*H-*imidazo[4,5-*b*]pyridin-6-carbonsäureamid;
3-(4-Methoxyphenyl)-*N*-(3-methoxypropyl)-3*H*-imidazo[4,5-*b*]pyridin-6-carbonsäureamid;
und deren pharmazeutisch unbedenklichen Salzen und N-Oxiden.

9. Zusammensetzung, enthaltend eine Verbindung nach einem der Ansprüche 1 bis 8 oder ein pharmazeutisch unbedenkliches Salz oder N-Oxid davon und einen pharmazeutisch unbedenklichen Träger.

10. Zusammensetzung, enthaltend eine Verbindung nach einem der Ansprüche 1 bis 8 oder ein pharmazeutisch unbedenkliches Salz oder N-Oxid davon und ein antineoplastisches Mittel, ein Antitumormittel, ein antiangiogenes Mittel oder ein chemotherapeutisches Mittel.

11. Zusammensetzung, enthaltend eine Verbindung nach einem der Ansprüche 1 bis 8 oder ein pharmazeutisch unbedenkliches Salz oder N-Oxid davon und ein cytotoxisches Mittel für die Krebstherapie.

12. Zusammensetzung, enthaltend eine Verbindung nach einem der Ansprüche 1 bis 8 oder ein pharmazeutisch unbedenkliches Salz oder N-Oxid davon und ein angiogenesehemmendes Mittel für die Krebstherapie.

13. Verbindungen nach einem der Ansprüche 1 bis 8 und deren pharmazeutisch umbedenkliche Salze und N-Oxide zur Verwendung bei der Behandlung einer hyperproliferativen Erkrankung.

14. Verbindungen nach einem der Ansprüche 1 bis 8 und deren pharmazeutisch unbedenkliche Salze und N-Oxide zur Verabreichung mit einem antineoplastischen Mittel, einem Antitumormittel, einem antiangiogenen Mittel oder einem chemotherapeutischen Mittel bei der Behandlung einer hyperproliferativen Erkrankung.

15. Verbindungen nach einem der Ansprüche 1 bis 8 und deren pharmazeutisch unbedenkliche Salze und N-Oxide zur Verwendung bei der Behandlung einer hyperproliferativen Erkrankung ausgewählt aus Brustkrebs, Kopfkrebs, Halskrebs, gastrointestinalem Krebs, Leukämie, Eierstockkrebs, Bronchialkrebs, Lungenkrebs oder Bauchspeicheldrüsenkrebs oder kleinzelligem Lungenkrebs und Kolonkrebs.

16. Verbindungen nach einem der Ansprüche 1 bis 8 und deren pharmazeutisch unbedenkliche Salze und N-Oxide zur Verwendung bei der Behandlung einer hyperproliferativen Erkrankung ausgewählt aus sinonasalem Natural-Killer/T-Zellen-Lymphom, Hodenkrebs (Seminom), Schilddrüsenkarzinom, malignem Melanom, adenoidcystischem Karzinom, Angiosarkom, anaplastischem großzelligem Lymphom, Endometriumkarzinom und Prostatakarzinom.

17. Verwendung einer Verbindung nach einem der Ansprüche 1 bis 8 oder eines pharmazeutisch unbedenklichen Salzes oder N-Oxids davon zur Herstellung eines Medikaments zur Behandlung einer wie in einem der Ansprüche 13, 15 oder 16 definierten hyperproliferativen Erkrankung.

## Revendications

1. Composé représenté par la Formule (I) 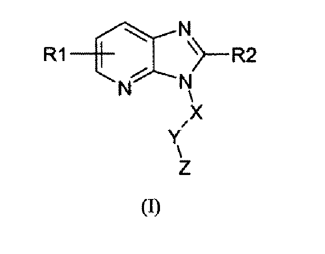 dans laquelle :
R1 est un groupe -CO-NR₃R₃₁ ;
R2 est un atome d'hydrogène, un groupe -C₀₋₈-alkyle ou un groupe -C₃₋₁₀-cycloalkyle ;
X est un groupe cyclyle ou un groupe hétérocyclyle éventuellement substitué par 1 ou plusieurs substituants choisis parmi un atome d'hydrogène, un atome d'halogène, un groupe NR₃₂R₃₃, un groupe NR₃₂COR₃₃, un groupe NR₃₂CO₂R₃₃, un groupe NR₃₂SO₂R₃₃, un groupe OR₃₂, un groupe SR₃₂, un groupe SO₂R₃₂, un groupe SO₂NR₃₂R₃₃, un groupe CO₂R₃₂, un groupe CO₂H, un groupe CONR₃₂R₃₃, un groupe -C₀₋₈-alkyle, un groupe -C₂₋₈-alcényle, un groupe -C₂₋₈-alcynyle, un groupe CN, un groupe CF₃, un groupe OCF₃, un groupe NO₂, un groupe oxo, un groupe cyclyle ou un groupe hétérocyclyle ;
Y est absent ou est un groupe 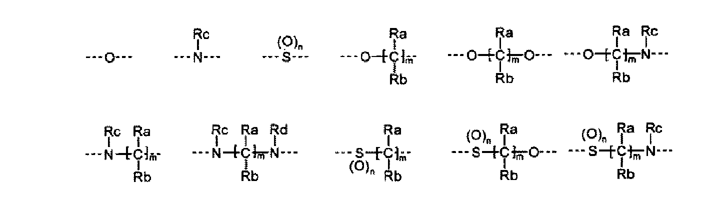 ou 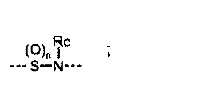 où le point de fixation à X peut être soit depuis la gauche, soit depuis la droite des groupes de liaison tels qu'illustrés ;
Rₐ et R_{b} sont chacun indépendamment un groupe -C₀₋₈-alkyle, un groupe -C₂₋₈-alcényle, un groupe -C₂₋₈-alcynyle, un groupe -C₃₋₁₀-cycloalkyle, un groupe -C₃₋₁₀-cycloalcényle, un groupe -C₁₋₈-alcoxy, un groupe -thio-C₁₋₈-alkyle, un groupe carboxyle, un groupe -N(C₀₋₈-alkyl)(C₀₋₈-alkyle), un groupe oxo ou un groupe hydroxy ; ou pris conjointement avec l'atome de carbone auquel ils sont fixés, forment un noyau saturé ou partiellement insaturé, à 3-10 chaînons, renfermant éventuellement de 0 à 4 atomes d'azote, d'oxygène, de soufre, ou groupes SO ou SO₂ aux noeuds du noyau ;
R_{c} et R_{d} sont chacun indépendamment un groupe -C₀₋₈-alkyle, un groupe -C₂₋₈-alcényle, un groupe benzyle ou un groupe acyle ; ou pris conjointement, ou avec Rₐ ou R_{b}, forment un noyau saturé ou partiellement insaturé, à 3-7 chaînons ;
m vaut 0, 1, 2, 3, 4 ou 5 ;
n vaut 0, 1 ou 2 ;
Z est un groupe cyclyle ou un groupe hétérocyclyle, éventuellement substitué par 1 ou plusieurs substituants choisis parmi un atome d'halogène, un groupe NR₃₄R₃₅, un groupe NR₃₄COR₃₅, un groupe NR₃₄CO₂R₃₅, un groupe NR₃₄SO₂R₃₅, un groupe OR₃₄, un groupe SR₃₄, un groupe SO₂R₃₄, un groupe SO₂NR₃₄R₃₅, un groupe CO₂R₃₄, un groupe CO₂H, un groupe CONR₃₄R₃₅, un groupe -C₀₋₈-alkyle, un groupe -C₂₋₈-alcényle, un groupe -C₂₋₈-alcynyle, un groupe CN, un groupe CF₃, un groupe NO₂, un groupe oxo, un groupe cyclyle ou un groupe hétérocyclyle ; ou, lorsque X et Y sont présents, Z peut être un groupe -C₁₋₈-alkyle ou un groupe -C₁₋₈-alkyl-O-C₁₋₈-alkyle ; et
R₃, R₃₁, R₃₂, R₃₃, R₃₄ et R₃₅ sont indépendamment un groupe alkyle en C₀₋₈ éventuellement substitué par un substituant hétérocyclyle ou OH ; un groupe -C₀₋₈-alkyl-C₃₋₈-cycloalkyle, un groupe CF₃, un groupe -C₀₋₈-alkyl-O-C₀₋₈-alkyle, un groupe -C₀₋₈-alkyl-N(C₀₋₈-alkyl)(C₀₋₈-alkyle), un groupe -C₀₋₈-alkyl-S(O)₀₋₂-C₀₋₈-alkyle ; ou un groupe hétérocyclyle éventuellement substitué par un substituant -C₀₋₈-alkyle, cyclyle ou cyclyle substitué ;
où les groupes cyclyle représentent des groupes cyclyle aromatiques, partiellement aromatiques ou non aromatiques, à 3-10 chaînons, et les groupes hétérocyclyle représentent des groupes hétérocyclyle aromatiques, partiellement aromatiques ou non aromatiques, à 3-10 chaînons, renfermant un ou plusieurs atomes choisis indépendamment parmi un atome d'azote, un atome d'oxygène et un atome de soufre, et leurs oxydes ;
ou un sel ou un N-oxyde pharmaceutiquement acceptable de celui-ci.

2. Composé selon la revendication 1, ou un sel ou un N-oxyde pharmaceutiquement acceptable de celui-ci, dans lequel X est un groupe cyclyle.

3. Composé selon la revendication 1 ou 2, ou un sel ou un N-oxyde pharmaceutiquement acceptable de celui-ci, dans lequel Y est absent.

4. Composé selon la revendication 1 ou 2, ou un sel ou un N-oxyde pharmaceutiquement acceptable de celui-ci, dans lequel Y est un groupe

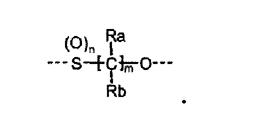

5. Composé selon la revendication 1 ou 2, ou un sel ou un N-oxyde pharmaceutiquement acceptable de celui-ci, dans lequel Y est un groupe

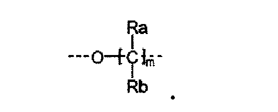

6. Composé selon la revendication 1 ou 2, ou un sel ou un N-oxyde pharmaceutiquement acceptable de celui-ci, dans lequel Y est un groupe

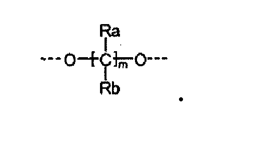

7. Composé selon la revendication 1 ou 2, ou un sel ou un N-oxyde pharmaceutiquement acceptable de celui-ci, dans lequel Y est un groupe

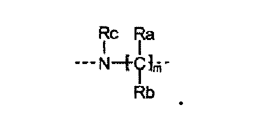

8. Composé constitué
du 3-{4-[(4-(trifluorométhoxy)benzyl)oxy]phényl}-*N*-(2-morpholin-4-yléthyl)-3*H*-imidazo[4,5-*b*]pyridine-6-carboxamide ;
du 3-{4-[(4-(trifluorométhoxy)benzyl)oxy]phényl}-*N*-(2-méthoxyéthyl)-3*H*-imidazo[4,5-*b*]pyridine-6-carboxamide ;
du 3-{3-[(4-(trifluorométhoxy)benzyl)oxy]phényl}-*N*-(pyridin-3-ylméthyl)-3*H*-imidazo[4,5-*b*]pyridine-6-carboxamide ;
du 3-{3-[(4-(trifluorométhoxy)benzyl)oxy]phényl}-*N*-(2-méthoxyéthyl)-3*H*-imidazo[4,5-*b*]pyridine-6-carboxamide ;
du 3-{4-[(4-(trifluorométhoxy)benzyl)oxy]phényl}-*N*-(3-(4-méthylpipérazin-1-yl)propyl)-3*H-*imidazo[4,5-*b*]pyridine-6-carboxamide ;
du 3-{4-[(4-(trifluorométhoxy)benzyl)oxy]phényl}-*N*-(2-diméthylaminoéthyl)-3*H*-imidazo[4,5-*b*]pyridine-6-carboxamide ;
du 3-{4-[(4-(trifluorométhoxy)benzyl)oxy]phényl}-*N*-(2-pipéridin-1-yléthyl)-3*H*-imidazo[4,5-*b*]pyridine-6-carboxamide ;
du 3-{4-[(4-(trifluorométhoxy)benzyl)oxy]phényl}-*N*-(pyridin-3-ylméthyl)-3*H*-imidazo[4,5-*b*]pyridine-6-carboxamide ;
du 3-{4-[(4-(trifluorométhoxy)benzyl)oxy]phényl}-*N*-méthyl-3*H*-imidazo[4,5-*b*]pyridine-6-carboxamide ;
du 3-{4-[(4-(trifluorométhoxy)benzyl)oxy]phényl}-*N*-éthyl-3*H*-imidazo[4,5-*b*]pyridine-6-carboxamide ;
du 3-{4-[(4-(trifluorométhoxy)benzyl)oxy]phényl}-*N*-(2-pyrrolidin-1-yléthyl)-3*H*-imidazo[4,5-*b*]pyridine-6-carboxamide ;
du 3-{3-[(4-(trifluorométhoxy)benzyl)oxy]phényl}-*N*-méthyl-3*H*-imidazo[4,5-*b*]pyridine-6-carboxamide ;
du 3-{3-[(4-(trifluorométhoxy)benzyl)oxy]phényl}-*N*-éthyl-3*H*-imidazo[4,5-*b*]pyridine-6-carboxamide ;
du 3-{3-[(4-(trifluorométhoxy)benzyl)oxy]phényl}-*N*-(3-méthoxypropyl)-3*H*-imidazo[4,5-*b*]pyridine-6-carboxamide ;
du 3-{3-[(4-(trifluorométhoxy)benzyl)oxy]phényl}-*N*-(2-diméthylaminoéthyl)-3*H*-imidazo[4,5-*b*]pyridine-6-carboxamide ;
du 3-(4-méthoxyphényl)-*N*-(pyridin-3-ylméthyl)-3*H-*imidazo[4,5-*b*]pyridine-6-carboxamide ;
du 3-(4-méthoxyphényl)-*N*-(2-morpholin-4-yléthyl)-3*H*-imidazo[4,5-*b*]pyridine-6-carboxamide ;
du 3-(4-méthoxyphényl)-*N*-(pyridin-2-ylméthyl)-3*H-*imidazo[4,5-*b*]pyridine-6-carboxamide ;
du 3-(4-méthoxyphényl)-*N*-(3-méthoxypropyl)-3*H-*imidazo[4,5-*b*]pyridine-6-carboxamide ;
ou un sel ou un N-oxyde pharmaceutiquement acceptable de celui-ci.

9. Composition comprenant un composé selon l'une quelconque des revendications 1 à 8, ou un sel ou un N-oxyde pharmaceutiquement acceptable de celui-ci, et un support pharmaceutiquement acceptable.

10. Composition comprenant un composé selon l'une quelconque des revendications 1 à 8, ou un sel ou un N-oxyde pharmaceutiquement acceptable de celui-ci ; et un agent anti-néoplasique, anti-tumoral, anti-angiogénique ou chimiothérapeutique.

11. Composition comprenant un composé selon l'une quelconque des revendications 1 à 8, ou un sel ou un N-oxyde pharmaceutiquement acceptable de celui-ci, et un agent cytotoxique thérapeutique contre le cancer.

12. Composition comprenant un composé selon l'une quelconque des revendications 1 à 8, ou un sel ou un N-oxyde pharmaceutiquement acceptable de celui-ci, et un agent thérapeutique contre le cancer, inhibant l'angiogénèse.

13. Composé selon l'une quelconque des revendications 1 à 8, ou un sel ou un N-oxyde pharmaceutiquement acceptable de celui-ci, destiné à être utilisé pour le traitement d'un trouble hyperprolifératif.

14. Composé selon l'une quelconque des revendications 1 à 8, ou un sel ou un N-oxyde pharmaceutiquement acceptable de celui-ci, destiné à être administré avec un agent anti-néoplasique, anti-tumoral, anti-angiogénique ou chimiothérapeutique pour le traitement d'un trouble hyperprolifératif.

15. Composé selon l'une quelconque des revendications 1 à 8, ou un sel ou un N-oxyde pharmaceutiquement acceptable de celui-ci, destiné à être utilisé pour le traitement d'un trouble hyperprolifératif choisi parmi un cancer du sein, un cancer de la tête, un cancer du cou, un cancer gastrointestinal, une leucémie, un cancer ovarien, bronchique, pulmonaire ou pancréatique, ou un cancer du côlon et du poumon à petites cellules.

16. Composé selon l'une quelconque des revendications 1 à 8, ou un sel ou un N-oxyde pharmaceutiquement acceptable de celui-ci, destiné à être utilisé pour le traitement d'un trouble hyperprolifératif choisi parmi un lymphome sinonasal à cellules tueuses naturelles/T, un cancer testiculaire (séminome), un carcinome de la thyroïde, un mélanome malin, un carcinome adénoïde cystique, un angiosarcome, un lymphome anaplasique à grandes cellules, un carcinome endométrial et un carcinome de la prostate.

17. Utilisation d'un composé selon l'une quelconque des revendications 1 à 8, ou d'un sel ou d'un N-oxyde pharmaceutiquement acceptable de celui-ci, pour la fabrication d'un médicament destiné au traitement d'un trouble hyperprolifératif tel que défini dans l'une quelconque des revendications 13, 15 ou 16.
